# EUROPÄISCHE PATENTSCHRIFT

(11) **EP 2 976 329 B1**
(45) Veröffentlichungstag und Bekanntmachung des Hinweises auf die Patenterteilung: **27.06.2018**
(21) Anmeldenummer: 14712595.9
(22) Anmeldetag: 04.03.2014
(51) Int. Cl.: C07D 219/02, C07D 401/10, C07D 403/04, C07D 403/14, C07D 413/10, C07D 241/16, C07D 487/04, C07D 265/10, C07D 491/04, C07D 279/36, C07D 209/82, H01L 51/50

(54) **MATERIALIEN FÜR ELEKTRONISCHE VORRICHTUNGEN**
MATERIALS FOR ELECTRONIC DEVICES
MATÉRIAUX POUR DISPOSITIFS ÉLECTRONIQUES

(30) Priorität: 22.03.2013 EP 13001486
(43) Veröffentlichungstag der Anmeldung: 27.01.2016
(60) Teilanmeldung: 18165596.0
(73) Patentinhaber: Merck Patent GmbH, 64293 Darmstadt (DE)
(72) Erfinder: STOESSEL, Philipp, 60487 Frankfurt am Main (DE); PARHAM, Amir, Hossain, 60486 Frankfurt am Main (DE); PFLUMM, Christof, 64291 Darmstadt-Arheiligen (DE); JATSCH, Anja, DE / 60489 Frankfurt am Main (DE)
(86) Internationale Anmeldenummer: PCT/EP2014/000537
(87) Internationale Veröffentlichungsnummer: WO 2014/146752

(56) Entgegenhaltungen:
- WO-A1-2012/143079
- WO-A1-2013/154064
- DE-T5-112006 002 147
- JP-A- 2004 178 896
- JP-A- 2004 288 381
- HENRY GILMAN, C. G. STUCKWISCH: "The di-metalation of 9-phenylcarbazole", J. AM. CHEM. SOC., Bd. 65, 1943, Seiten 1729-1733, XP002723834,
- TOSHIKAZU KITAGAWA, MAKOTO NISHIMURA, KENICHI TAKEUCHI, KUNIO OKAMOTO: "Bis(2,6-diluorophenyl)benzoylmethyl cation: alpha-ketocarbenium ion as a single-electron acceptor", TETRAHEDRON LETTERS, Bd. 32, Nr. 27, 1991, Seiten 3187-3190, XP002723835,
- HIROKI UOYAMA ET AL: "Highly efficient organic light-emitting diodes from delayed fluorescence", NATURE, vol. 492, no. 7428, 12 December 2012 (2012-12-12), pages 234-238, XP055048388, ISSN: 0028-0836, DOI: 10.1038/nature11687

## Beschreibung

Die vorliegende Anmeldung betrifft eine Verbindung einer Formel (I), welche eine Benzolgruppe aufweist, die mit einer Gruppe gewählt aus Carbazolderivaten und verbrückten Aminen und mit einer elektronenziehenden Gruppe substituiert ist, wobei die beiden Gruppen in ortho-Position zueinander stehen. Die Verbindung kann in einer elektronischen Vorrichtung verwendet werden.

Unter elektronischen Vorrichtungen im Sinne dieser Anmeldung werden insbesondere sogenannte organische elektronische Vorrichtungen verstanden (organic electronic devices), welche organische Halbleitermaterialien als Funktionsmaterialien enthalten. Nochmals insbesondere werden darunter organische Elektrolumineszenzvorrichtungen (OLEDs) und andere elektronische Vorrichtungen verstanden, welche im Folgenden bei der detaillierten Beschreibung der Erfindung aufgeführt sind.

Allgemein wird unter der Bezeichnung OLED eine elektronische Vorrichtung verstanden, welche mindestens ein organisches Material enthält und unter Anlegen von elektrischer Spannung Licht emittiert. Der genaue Aufbau von OLEDs ist unter anderem in US 4539507, US 5151629, EP 0676461 und WO 98/27136 beschrieben.

Bei elektronischen Vorrichtungen, insbesondere OLEDs, besteht großes Interesse an der Verbesserung der Leistungsdaten, insbesondere Lebensdauer und Effizienz und Betriebsspannung. Eine wichtige Rolle spielen dabei organische Emitterschichten, insbesondere die darin enthaltenen Emitterverbindungen.

Zur Lösung dieser technischen Aufgabe werden kontinuierlich neue Materialien gesucht, die sich zur Verwendung als Emitterverbindungen in emittierenden Schichten eignen, insbesondere in Kombination mit einem Matrixmaterial.

Unter einem Matrixmaterial wird in einem System enthaltend zwei oder mehr Materialien diejenige Komponente verstanden, deren Anteil in der Mischung der größere ist. Entsprechend wird unter einem Dotanden in einem System enthaltend zwei oder mehr Materialien diejenige Komponente verstanden, deren Anteil in der Mischung der kleinere ist.

Als Emitterverbindungen einer emittierenden Schicht gelten im Rahmen der vorliegenden Anmeldung Verbindungen, welche bei Betrieb der elektronischen Vorrichtung Licht emittieren.

In emittierenden Schichten von OLEDs sind im Allgemeinen die Dotandverbindung bzw. die Dotandverbindungen die emittierenden Verbindungen, und die Matrixverbindung bzw. die Matrixverbindungen sind nicht lichtemittierend. Es können jedoch auch Ausnahmen vorliegen, beispielsweise Verbindungen, welche in geringem Anteil in der Mischung der lichtemittierenden Schicht vorhanden sind, also gemäß obenstehender Definition als Dotanden gelten, jedoch nicht emittieren, sondern andere Aufgaben erfüllen, beispielsweise Ladungstransport.

Im Stand der Technik bekannt sind Verbindungen enthaltend eine oder mehrere Carbazolgruppen, beispielsweise aus WO 2005/039246, US 2005/0069729, JP 2004/288381, EP 1205527 oder WO 2008/086851. Die Verbindungen eignen sich unter anderem zur Verwendung als Matrixmaterialien für emittierende Schichten von OLEDs oder als Verbindungen für elektronentransportierende Schichten von OLEDs.

Weiterhin sind aus der WO 2012/143079 Verbindungen bekannt, welche drei Carbazolgruppen in 1, 3, 5-Position an einem Benzol aufweisen. Weitere Substitutenten sind dann beispielsweise gewählt aus Alkylgruppen. Die Verbindungen eignen sich unter anderem zur Verwendung in der emittierenden Schicht von OLEDs.

Nochmals weiterhin sind in H. Uoyama et al., Nature 2012, 492, 234 ff., Verbindungen offenbart, welche mehrere Carbazolgruppen und mehrere Cyanogruppen gebunden an einen Benzolring enthalten, wobei die Carbazolgruppen und die Cyanogruppen in einer definierten Anordnung zueinander stehen. Dabei sind grundsätzlich mindestens zwei Carbazolgruppen zueinander para am Benzolring angeordnet. Die Verbindungen werden in der emittierenden Schicht von OLEDs verwendet.

Trotz dieser Ergebnisse besteht weiterhin Bedarf an neuen Verbindungen, die sich zur Verwendung in elektronischen Vorrichtungen, insbesondere zur Verwendung in emittierenden Schichten von OLEDs eignen.

Es wurde nun überraschend gefunden, dass sich Verbindungen, welche mindestens ein Carbazolderivat an einen Benzolring gebunden aufweisen und in ortho-Position dazu mindestens eine elektronenziehende Gruppe, wobei die Substituenten am Benzolring in einer definierten Anordnung, wie definiert in untenstehender Formel (I), zueinander stehen, sehr gut zur Verwendung in elektronischen Vorrichtungen eignen. Insbesondere eignen sie sich hervorragend zur Verwendung als Emitterverbindungen in emittierenden Schichten.

OLEDs enthaltend die Verbindungen weisen überraschenderweise eine sehr gute Leistungseffizienz und eine sehr lange Lebensdauer auf. Weiterhin weisen sie bevorzugt eine niedrige Betriebsspannung auf. Nochmals weiterhin kann mit ihnen bei Verwendung als Emitterverbindung potentiell das gesamte Farbspektrum der Emission abgedeckt werden.

Gegenstand der vorliegenden Anmeldung ist somit eine Verbindung gemäß Anspruch 1.

Unter heteroaromatischen Ringsystemen, welche über ein RingStickstoffatom gebunden sind, werden insbesondere Derivate des Carbazols, Indenocarbazols, Indolocarbazols, Pyrrols und Imidazols verstanden, die über ihr entsprechendes Stickstoffatom mit freier Valenz gebunden sind.

Eine Arylgruppe im Sinne dieser Erfindung enthält 6 bis 60 aromatische Ringatome; eine Heteroarylgruppe im Sinne dieser Erfindung enthält 5 bis 60 aromatische Ringatome, von denen mindestens eines ein Heteroatom darstellt. Die Heteroatome sind bevorzugt ausgewählt aus N, O und S. Dies stellt die grundlegende Definition dar. Werden in der Beschreibung der vorliegenden Erfindung andere Bevorzugungen angegeben, beispielsweise bezüglich der Zahl der aromatischen Ringatome oder der enthaltenen Heteroatome, so gelten diese.

Dabei wird unter einer Arylgruppe bzw. Heteroarylgruppe entweder ein einfacher aromatischer Cyclus, also Benzol, bzw. ein einfacher heteroaromatischer Cyclus, beispielsweise Pyridin, Pyrimidin oder Thiophen, oder ein kondensierter (annellierter) aromatischer bzw. heteroaromatischer Polycyclus, beispielsweise Naphthalin, Phenanthren, Chinolin oder Carbazol verstanden. Ein kondensierter (annellierter) aromatischer bzw. heteroaromatischer Polycyclus besteht im Sinne der vorliegenden Anmeldung aus zwei oder mehr miteinander kondensierten einfachen aromatischen bzw. heteroaromatischen Cyclen.

Unter einer Aryl- oder Heteroarylgruppe, die jeweils mit den oben genannten Resten substituiert sein kann und die über beliebige Positionen am Aromaten bzw. Heteroaromaten verknüpft sein kann, werden insbesondere Gruppen verstanden, welche abgeleitet sind von Benzol, Naphthalin, Anthracen, Phenanthren, Pyren, Dihydropyren, Chrysen, Perylen, Fluoranthen, Benzanthracen, Benzphenanthren, Tetracen, Pentacen, Benzpyren, Furan, Benzofuran, Isobenzofuran, Dibenzofuran, Thiophen, Benzothiophen, Isobenzothiophen, Dibenzothiophen, Pyrrol, Indol, Isoindol, Carbazol, Pyridin, Chinolin, Isochinolin, Acridin, Phenanthridin, Benzo-5,6-chinolin, Benzo-6,7-chinolin, Benzo-7,8-chinolin, Phenothiazin, Phenoxazin, Pyrazol, Indazol, Imidazol, Benzimidazol, Naphthimidazol, Phenanthrimidazol, Pyridimidazol, Pyrazinimidazol, Chinoxalinimidazol, Oxazol, Benzoxazol, Naphthoxazol, Anthroxazot, Phenanthroxazol, Isoxazol, 1,2-Thiazol, 1,3-Thiazol, Benzothiazol, Pyridazin, Benzopyridazin, Pyrimidin, Benzpyrimidin, Chinoxalin, Pyrazin, Phenazin, Naphthyridin, Azacarbazol, Benzocarbolin, Phenanthrolin, 1,2,3-Triazol, 1,2,4-Triazol, Benzotriazol, 1,2,3-Oxadiazol, 1,2,4-Oxadiazol, 1,2,5-Oxadiazol, 1,3,4-Oxadiazol, 1,2,3-Thiadiazol, 1,2,4-Thiadiazol, 1,2,5-Thiadiazol, 1,3,4-Thiadiazol, 1,3,5-Triazin, 1,2,4-Triazin, 1,2,3-Triazin, Tetrazol, 1,2,4,5-Tetrazin, 1,2,3,4-Tetrazin, 1,2,3,5-Tetrazin, Purin, Pteridin, Indolizin und Benzothiadiazol.

Ein aromatisches Ringsystem im Sinne dieser Erfindung enthält 6 bis 60 C-Atome im Ringsystem. Ein heteroaromatisches Ringsystem im Sinne dieser Erfindung enthält 5 bis 60 aromatische Ringatome, von denen mindestens eines ein Heteroatom darstellt. Die Heteroatome sind bevorzugt ausgewählt aus N, O und/oder S. Unter einem aromatischen oder heteroaromatischen Ringsystem im Sinne dieser Erfindung soll ein System verstanden werden, das nicht notwendigerweise nur Aryl- oder Heteroarylgruppen enthält, sondern in dem auch mehrere Aryl- oder Heteroarylgruppen durch eine nicht-aromatische Einheit (bevorzugt weniger als 10 % der von H verschiedenen Atome), wie z. B. ein sp³-hybridisiertes C-, Si-, N- oder O-Atom, ein sp²-hybridisiertes C- oder N-Atom oder ein sp-hybridisiertes C-Atom, verbunden sein können. So sollen beispielsweise auch Systeme wie 9,9'-Spirobifluoren, 9,9'-Diarylfluoren, Triarylamin, Diarylether, Stilben, etc. als aromatische Ringsysteme im Sinne dieser Erfindung verstanden werden, und ebenso Systeme, in denen zwei oder mehrere Arylgruppen beispielsweise durch eine lineare oder cyclische Alkyl-, Alkenyl- oder Alkinylgruppe oder durch eine Silylgruppe verbunden sind. Weiterhin werden auch Systeme, in denen zwei oder mehr Aryl- oder Heteroarylgruppen über Einfachbindungen miteinander verknüpft sind, als aromatische oder heteroaromatische Ringsysteme im Sinne dieser Erfindung verstanden, wie beispielsweise Systeme wie Biphenyl, Terphenyl oder Diphenyltriazin.

Unter einem aromatischen oder heteroaromatischen Ringsystem mit 5 - 60 aromatischen Ringatomen, welches noch jeweils mit Resten wie oben definiert substituiert sein kann und welches über beliebige Positionen am Aromaten bzw. Heteroaromaten verknüpft sein kann, werden insbesondere Gruppen verstanden, die abgeleitet sind von Benzol, Naphthalin, Anthracen, Benzanthracen, Phenanthren, Benzphenanthren, Pyren, Chrysen, Perylen, Fluoranthen, Naphthacen, Pentacen, Benzpyren, Biphenyl, Biphenylen, Terphenyl, Terphenylen, Quaterphenyl, Fluoren, Spirobifluoren, Dihydrophenanthren, Dihydropyren, Tetrahydropyren, cis- oder trans-Indenofluoren, Truxen, Isotruxen, Spirotruxen, Spiroisotruxen, Furan, Benzofuran, Isobenzofuran, Dibenzofuran, Thiophen, Benzothiophen, Isobenzothiophen, Dibenzothiophen, Pyrrol, Indol, Isoindol, Carbazol, Indolocarbazol, Indenocarbazol, Pyridin, Chinolin, Isochinolin, Acridin, Phenanthridin, Benzo-5,6-chinolin, Benzo-6,7-chinolin, Benzo-7,8-chinolin, Phenothiazin, Phenoxazin, Pyrazol, Indazol, Imidazol, Benzimidazol, Naphthimidazol, Phenanthrimidazol, Pyridimidazol, Pyrazinimidazol, Chinoxalinimidazol, Oxazol, Benzoxazol, Naphthoxazol, Anthroxazol, Phenanthroxazol, Isoxazol, 1,2-Thiazol, 1,3-Thiazol, Benzothiazol, Pyridazin, Benzopyridazin, Pyrimidin, Benzpyrimidin, Chinoxalin, 1,5-Diazaanthracen, 2,7-Diazapyren, 2,3-Diazapyren, 1,6-Diazapyren, 1,8-Diazapyren, 4,5-Diazapyren, 4,5,9,10-Tetraazaperylen, Pyrazin, Phenazin, Phenoxazin, Phenothiazin, Fluorubin, Naphthyridin, Azacarbazol, Benzocarbolin, Phenanthrolin, 1,2,3-Triazol, 1,2,4-Triazol, Benzotriazol, 1,2,3-Oxadiazol, 1,2,4-Oxadiazol, 1,2,5-Oxadiazol, 1,3,4-Oxadiazol, 1,2,3-Thiadiazol, 1,2,4-Thiadiazol, 1,2,5-Thiadiazol, 1,3,4-Thiadiazol, 1,3,5-Triazin, 1,2,4-Triazin, 1,2,3-Triazin, Tetrazol, 1,2,4,5-Tetrazin, 1,2,3,4-Tetrazin, 1,2,3,5-Tetrazin, Purin, Pteridin, Indolizin und Benzothiadiazol oder Kombinationen dieser Gruppen.

Im Rahmen der vorliegenden Erfindung werden unter einer geradkettigen Alkylgruppe mit 1 bis 40 C-Atomen bzw. einer verzweigten oder cyclischen Alkylgruppe mit 3 bis 40 C-Atomen bzw. einer Alkenyl- oder Alkinylgruppe mit 2 bis 40 C-Atomen, in der auch einzelne H-Atome oder CH₂-Gruppen durch die oben bei der Definition der Reste genannten Gruppen substituiert sein können, bevorzugt die Reste Methyl, Ethyl, n-Propyl, i-Propyl, n-Butyl, i-Butyl, s-Butyl, t-Butyl, 2-Methylbutyl, n-Pentyl, s-Pentyl, Cyclopentyl, neoPentyl, n-Hexyl, Cyclohexyl, neo-Hexyl, n-Heptyl, Cycloheptyl, n-Octyl, Cyclooctyl, 2-Ethylhexyl, Trifluormethyl, Pentafluorethyl, 2,2,2-Trifluorethyl, Ethenyl, Propenyl, Butenyl, Pentenyl, Cyclopentenyl, Hexenyl, Cyclohexenyl, Heptenyl, Cycloheptenyl, Octenyl, Cyclooctenyl, Ethinyl, Propinyl, Butinyl, Pentinyl, Hexinyl oder Octinyl verstanden. Unter einer Alkoxy- oder Thioalkylgruppe mit 1 bis 40 C-Atomen werden bevorzugt Methoxy, Trifluormethoxy, Ethoxy, n-Propoxy, i-Propoxy, n-Butoxy, i-Butoxy, s-Butoxy, t-Butoxy, n-Pentoxy, s-Pentoxy, 2-Methylbutoxy, n-Hexoxy, Cyclohexyloxy, n-Heptoxy, Cycloheptyloxy, n-Octyloxy, Cyclooctyloxy, 2-Ethylhexyloxy, Pentafluorethoxy, 2,2,2-Trifluorethoxy, Methylthio, Ethylthio, n-Propylthio, i-Propylthio, n-Butylthio, i-Butylthio, s-Butylthio, t-Butylthio, n-Pentylthio, s-Pentylthio, n-Hexylthio, Cyclohexylthio, n-Heptylthio, Cycloheptylthio, n-Octylthio, Cyclooctylthio, 2-Ethylhexylthio, Trifluormethylthio, Pentafluorethylthio, 2,2,2-Trifluorethylthio, Ethenylthio, Propenylthio, Butenylthio, Pentenylthio, Cyclopentenylthio, Hexenylthio, Cyclohexenylthio, Heptenylthio, Cycloheptenylthio, Octenylthio, Cyclooctenylthio, Ethinylthio, Propinylthio, Butinylthio, Pentinylthio, Hexinylthio, Heptinylthio oder Octinylthio verstanden.

Unter der Formulierung, dass zwei oder mehr Reste miteinander einen Ring bilden können, soll im Rahmen der vorliegenden Anmeldung unter anderem verstanden werden, dass die beiden Reste miteinander durch eine chemische Bindung verknüpft sind. Dies wird durch das folgende Schema verdeutlicht:

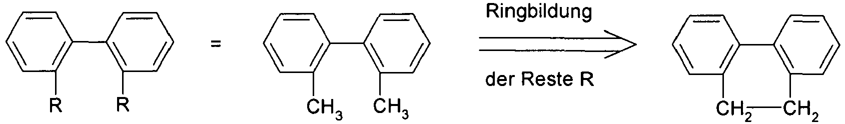

Weiterhin soll unter der oben genannten Formulierung aber auch verstanden werden, dass für den Fall, dass einer der beiden Reste Wasserstoff darstellt, der zweite Rest unter Bildung eines Rings an die Position, an die das Wasserstoffatom gebunden war, bindet. Dies soll durch das folgende Schema verdeutlicht werden:

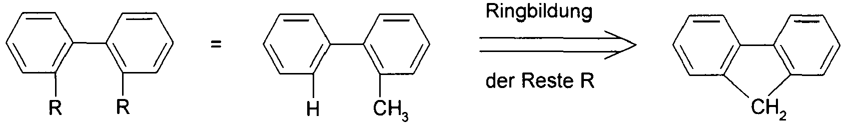

Formel (A) entspricht bevorzugt einer der folgenden Formeln (A-1) bis (A-22), die über die gestrichelte Bindung gebunden sind 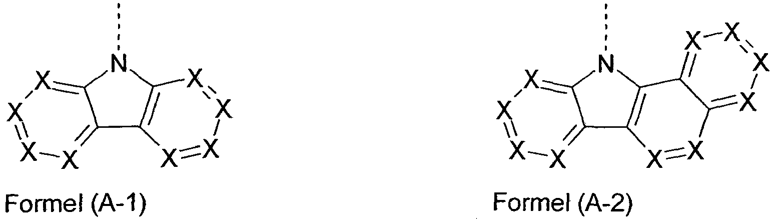 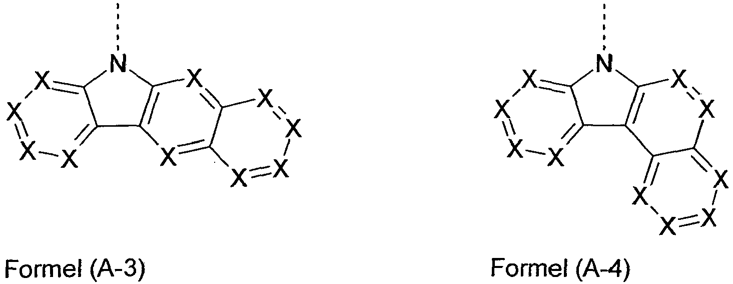 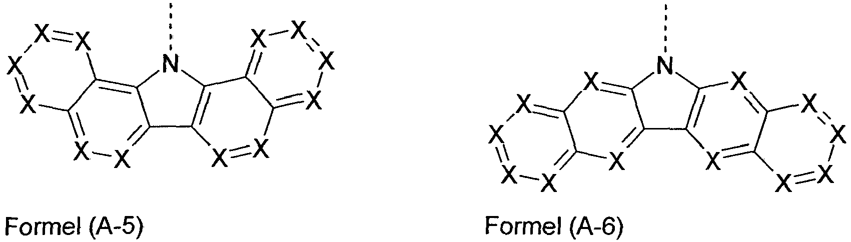 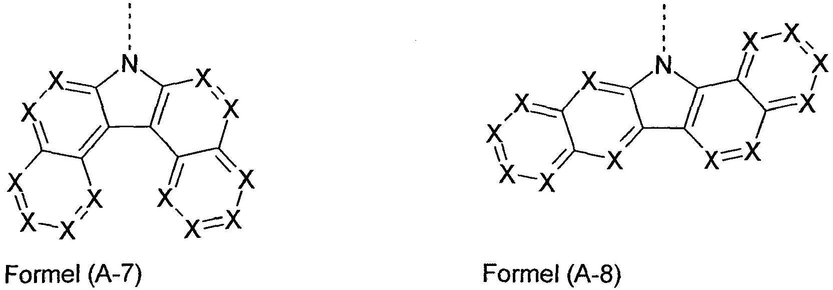 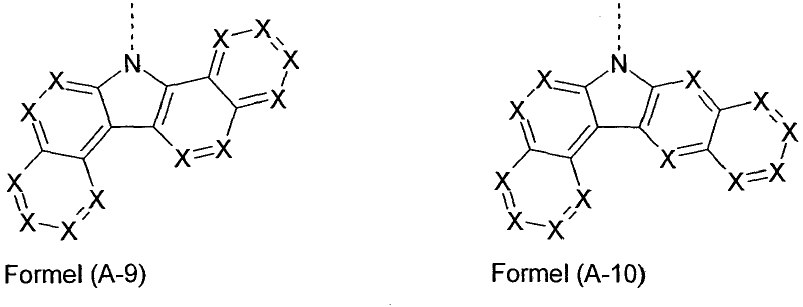 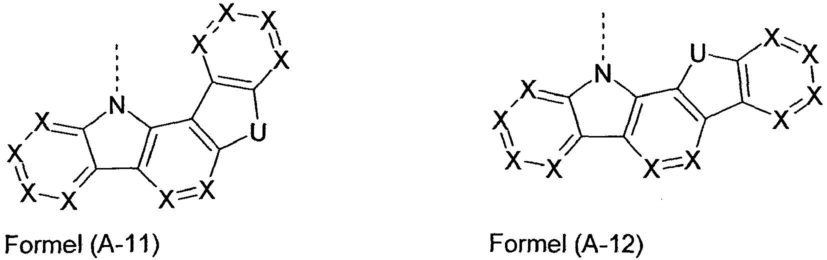 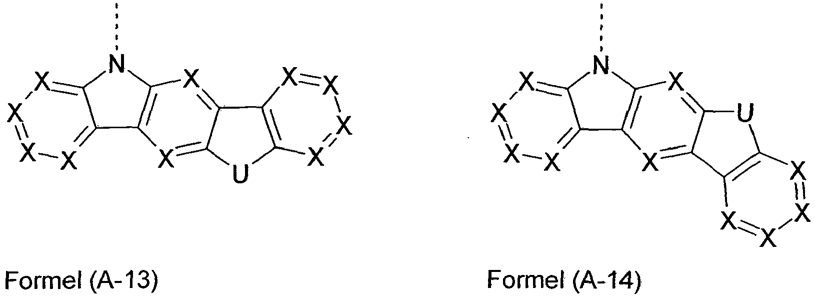 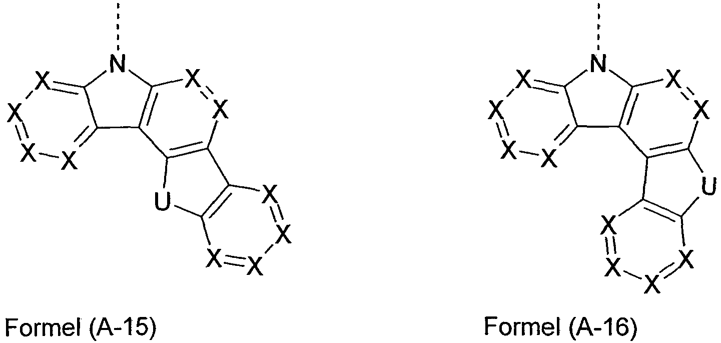 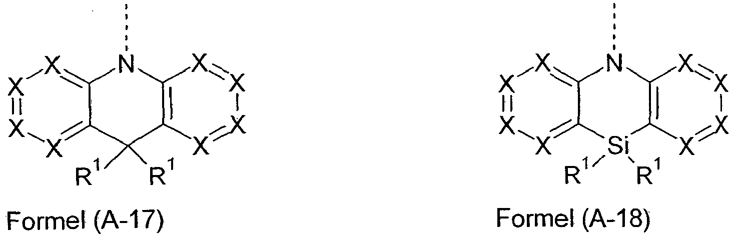 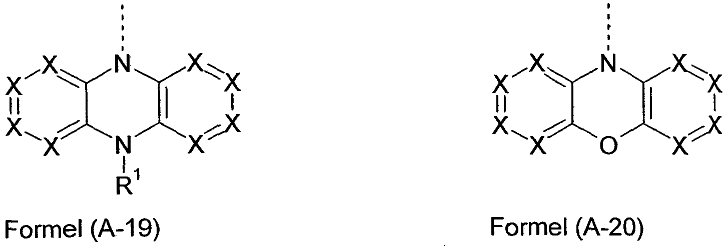 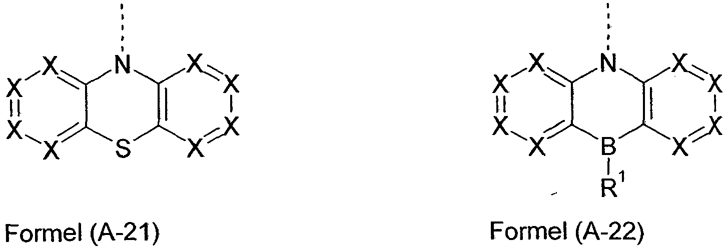 wobei gilt:
- X: ist bei jedem Auftreten gleich oder verschieden CR¹ oder N;
- U: ist gleich BR¹, C(R¹)₂, Si(R¹)₂, NR¹, O oder S; und
- R¹: ist definiert wie oben.

Bevorzugt unter den Formeln (A-1) bis (A-22) sind die Formeln (A-1), (A-4), (A-14) und (A-17).

Wenn U für eine Gruppe C(R¹)₂ oder Si(R¹)₂ steht, ist es bevorzugt, dass die beiden Gruppen R¹ der Gruppe miteinander verknüpft sind und einen Ring bilden. Bevorzugt wird dabei ein fünf- oder sechsgliedriger Ring gebildet. Besonders bevorzugt entsteht dabei eine Spiro-Bifluoren-Gruppe.

Es ist für die obenstehenden Formeln bevorzugt, dass X gleich CR¹ ist.

Es ist weiterhin bevorzugt für die obenstehenden Formeln, dass nicht mehr als drei Gruppen X pro Ring gleich N sind. Weiterhin ist es bevorzugt, dass nicht mehr als zwei benachbarte Gruppen X in einem Ring gleich N sind. Besonders bevorzugt ist genau eine Gruppe X pro Ring gleich N, oder es ist keine Gruppe X pro Ring gleich N.

Bevorzugt ist die Gruppe U in obenstehenden Formeln gleich C(R¹)₂ oder NR¹, besonders bevorzugt gleich C(R¹)₂.

Besonders bevorzugt sind Kombinationen der oben genannten bevorzugten Ausführungsformen miteinander.

Bevorzugt ist Ar¹ bei jedem Auftreten gleich oder verschieden ein aromatisches Ringsystem mit 6 bis 20 aromatischen Ringatomen, das mit einem oder mehreren Resten R¹ substituiert sein kann, und besonders bevorzugt Phenyl, Pyridyl, Naphthyl, Fluorenyl, oder Carbazolyl, die jeweils mit einem oder mehreren Resten R¹ substituiert sein können.

Bevorzugt ist die Gruppe Y bei jedem Auftreten gleich oder verschieden eine Einfachbindung, C(R¹)₂, NR¹, O oder S, besonders bevorzugt eine Einfachbindung.

Es ergibt sich aus der Definition von R^{A} und R^{B}, dass diese kein über das Stickstoffatom gebundenes Carbazol oder Carbazolderivat sein dürfen.

Es ist bevorzugt, dass mindestens zwei Gruppen V in den Gruppen E vorliegen, besonders bevorzugt zwei, drei, vier oder fünf.

Die Gruppe E ist bevorzugt eine Gruppe der Formel (E-1) bis (E-9), die über die gestrichelte Bindung gebunden ist 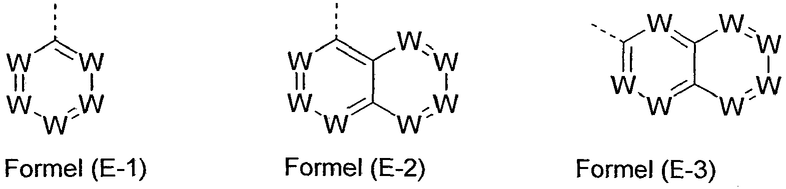 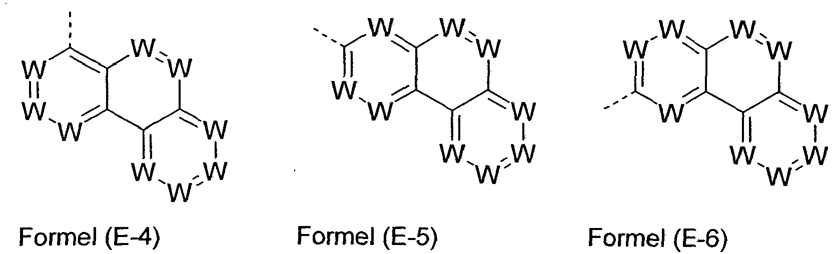 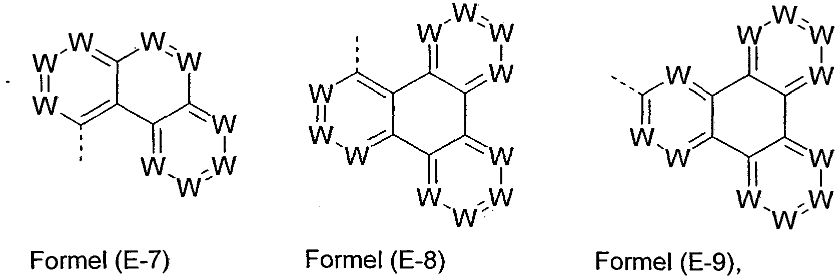 wobei gilt:
- W: ist bei jedem Auftreten gleich oder verschieden CR¹ oder V, wobei mindestens eine Gruppe W gleich V ist; und
wobei V und R¹ definiert sind wie oben.

Bevorzugt unter den Gruppen der Formeln (E-1) bis (E-9) ist die Gruppe der Formel (E-1).

Bevorzugt sind in den Gruppen der Formel (E-1) bis (E-9) mindestens zwei Gruppen W gleich V, besonders bevorzugt genau zwei, drei, vier oder fünf.

Weiterhin ist es bevorzugt, dass nicht mehr als zwei Gruppen V, die gleich =N- sind, nebeneinander in einem Ring vorliegen. Weiterhin bevorzugt liegen nicht mehr als drei Gruppen V, die gleich =N- sind, in einem Ring vor.

Besonders bevorzugte Ausführungsformen der Gruppen E entsprechen den folgenden Formeln (E-1-1) bis (E-1-89)

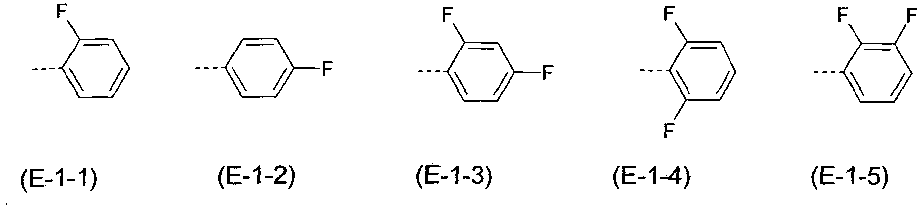

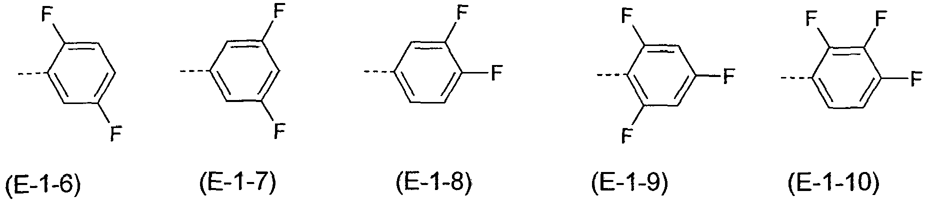

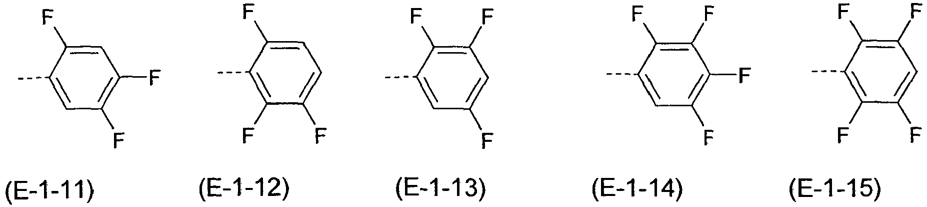

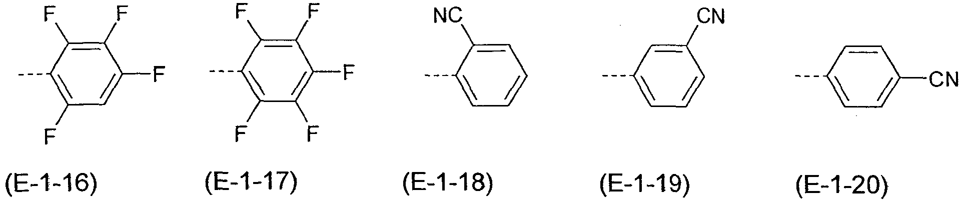

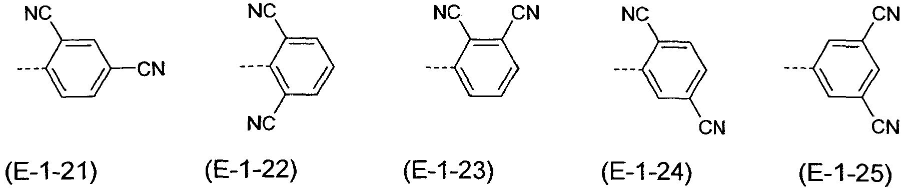

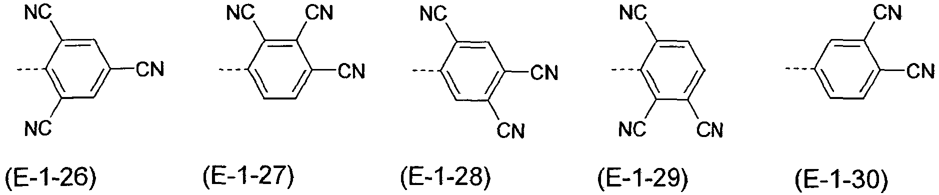

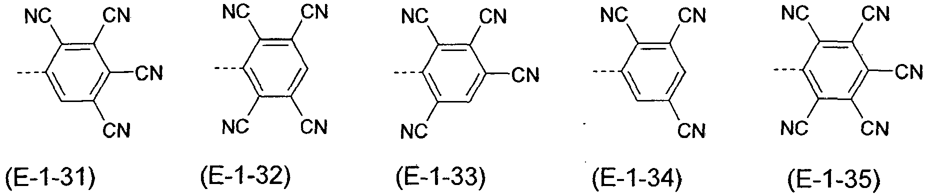

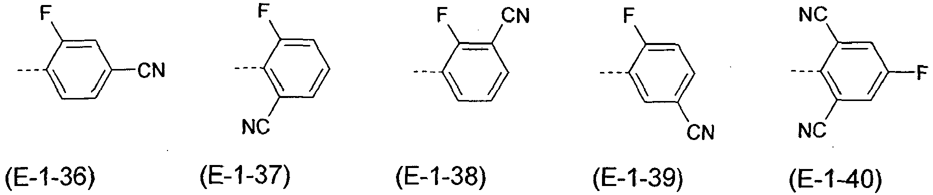

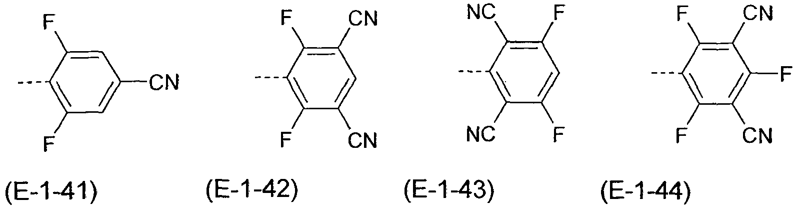

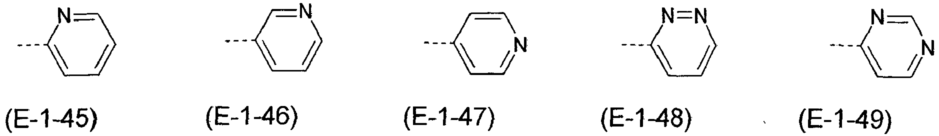

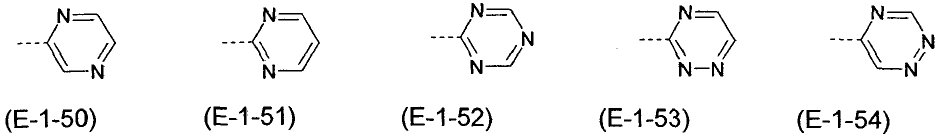

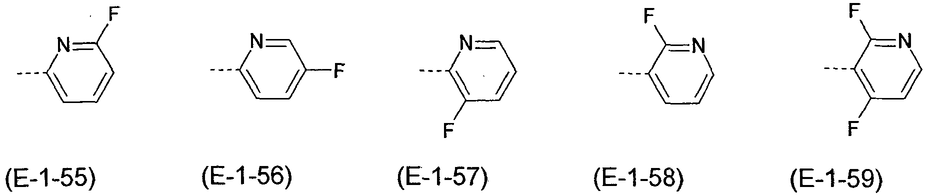

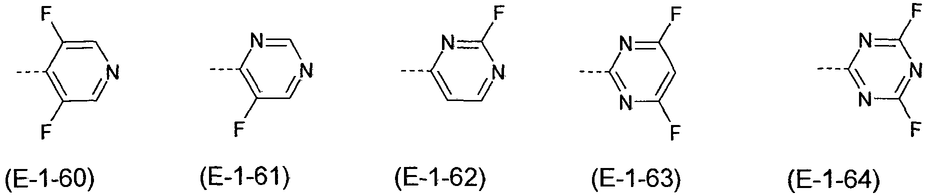

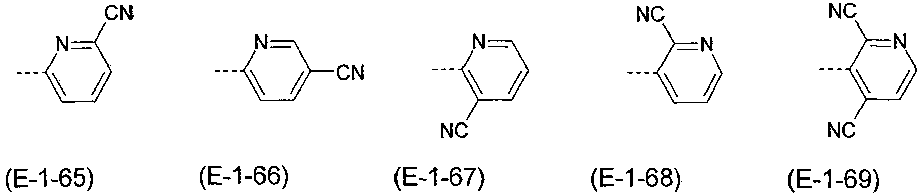

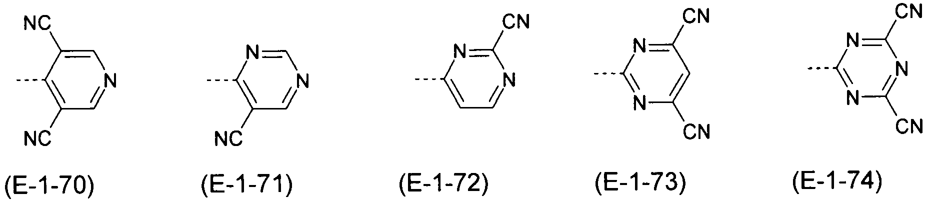

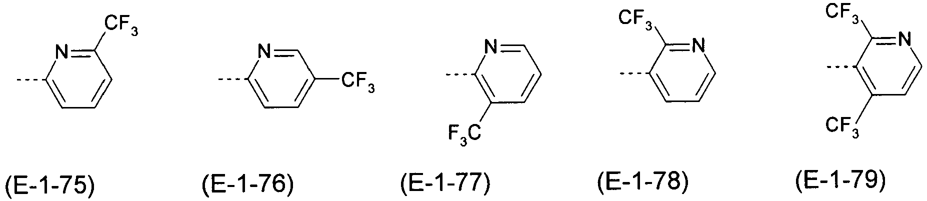

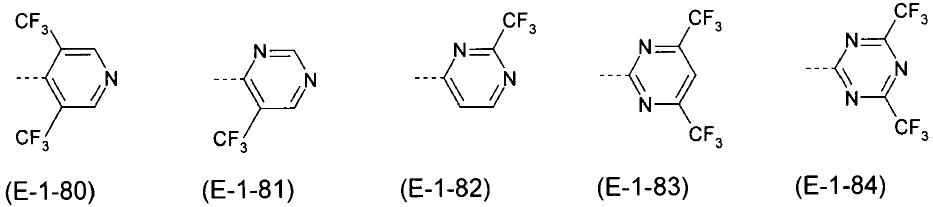

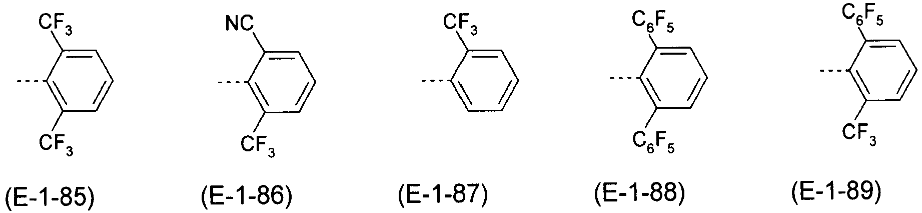

Bevorzugt ist der Rest R¹ bei jedem Auftreten gleich oder verschieden H, D, F, CN, Si(R²)₃, N(R²)₂, eine geradkettige Alkyl- oder Alkoxygruppe mit 1 bis 10 C-Atomen oder eine verzweigte oder cyclische Alkyl- oder Alkoxygruppe mit 3 bis 10 C-Atomen, wobei die oben genannten Gruppen jeweils mit einem oder mehreren Resten R² substituiert sein können und wobei in den oben genannten Gruppen eine oder mehrere CH₂-Gruppen durch -R²C=CR²-, -C≡C-, Si(R²)₂, C=O, C=NR², -C(=O)O-, -C(=O)NR²-, NR², P(=O)(R²), -O-, -S-, SO oder SO₂ ersetzt sein können, oder ein aromatisches oder heteroaromatisches Ringsystem mit 5 bis 20 aromatischen Ringatomen, das jeweils mit einem oder mehreren Resten R² substituiert sein kann, wobei zwei oder mehr Reste R¹ miteinander verknüpft sein können und einen Ring bilden können.

Bevorzugte Ausführungsformen der Verbindungen der Formel (I) entsprechen den Formeln (I-2) oder (I-3) 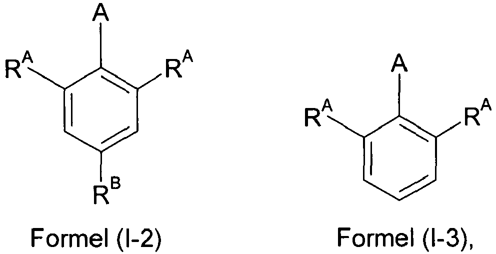 wobei an die freien Positionen am Benzolring in Formel (I-2) wahlweise H, Alkylgruppen mit 1 bis 10 C-Atomen oder Arylgruppen mit 6 bis 14 aromatischen Ringatomen gebunden sein können, die jeweils mit einem oder mehreren Resten R¹ substituiert sein können, und wobei die Gruppen A, R^{A} und R^{B} gemäß Anspruch 1 definiert sind.

Weiterhin bevorzugt ist die Kombination der bevorzugten Ausführungsformen von insbesondere A, B und R^{A} und R^{B} mit den Formeln (I-2) und (I-3).

Insbesondere bevorzugt entspricht in den Formeln (I-2) und (I-3) die Gruppe A einer der Formeln (A-1) bis (A-22), wie oben definiert.

Besonders bevorzugte Ausführungsformen der Verbindungen der Formel (I) sind die Verbindungen der Formel (I-2-a) bis (I-2-f) 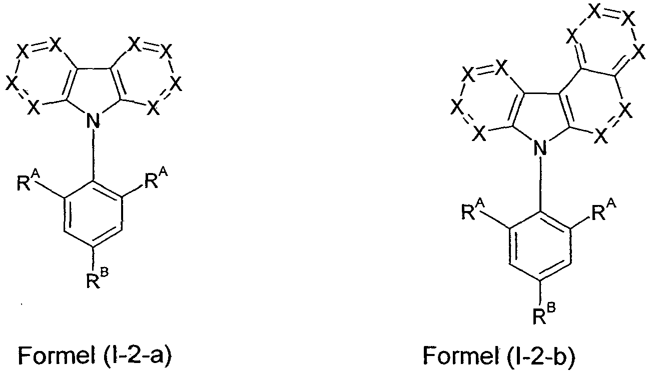 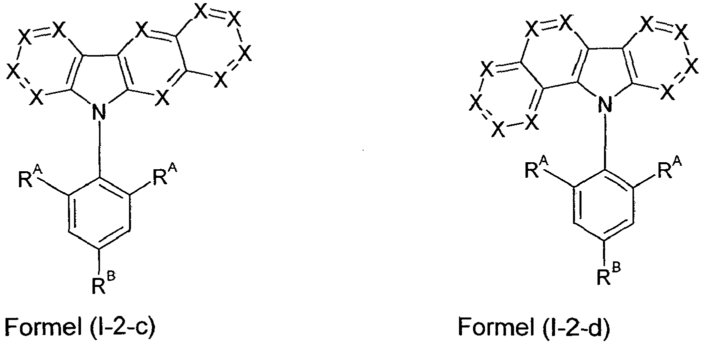 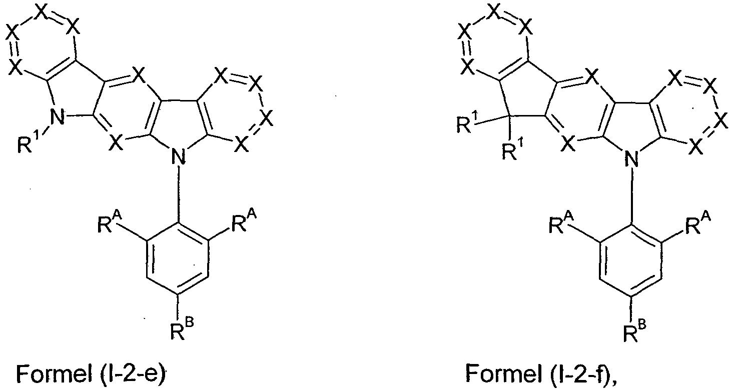 wobei an die freien Positionen am Benzolring in Formeln (I-2-1) bis (I-2-f) wahlweise H, Alkylgruppen mit 1 bis 10 C-Atomen oder Arylgruppen mit 6 bis 14 aromatischen Ringatomen gebunden sein können, die jeweils mit einem oder mehreren Resten R¹ substituiert sein können, und
wobei gilt:
X ist definiert wie oben und ist bevorzugt gleich CR¹;
R^{A} und R^{B} sind definiert wie in Anspruch 1.

Besonders bevorzugte Ausführungsformen der Verbindungen der Formel (I) sind die Verbindungen der Formel (I-3-a) bis (I-3-f) 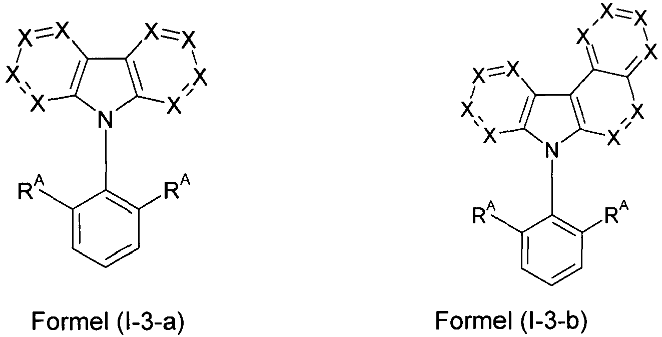 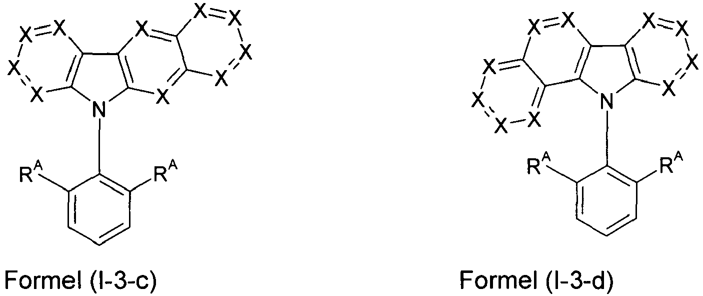 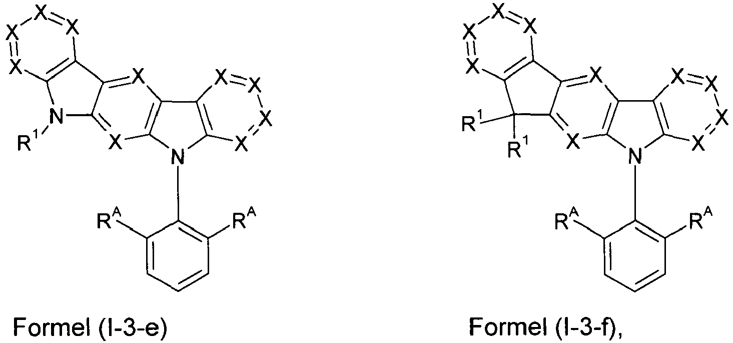 wobei gilt:
X ist definiert wie oben und ist bevorzugt gleich CR¹;
R^{A} ist definiert wie in Anspruch 1.

Beispiele für Verbindungen gemäß der vorliegenden Erfindung sind im Folgenden abgebildet:

| | | |
|---|---|---|
| 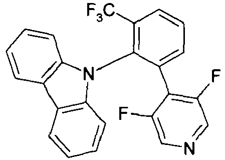 | 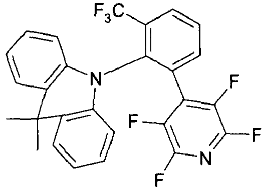 | 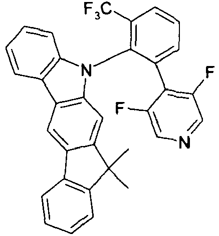 |
| 1 | 2 | 3 |
| 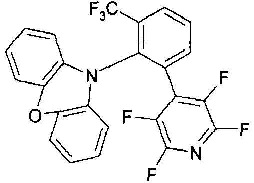 | 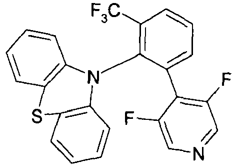 | 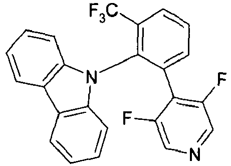 |
| 4 | 5 | 6 |
| 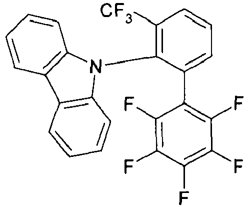 | 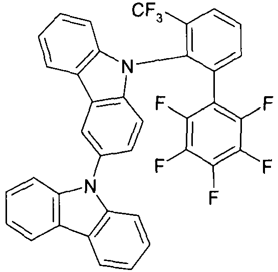 | 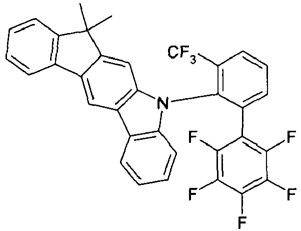 |
| 7 | 8 | 9 |
| 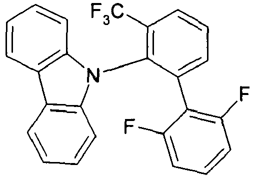 | 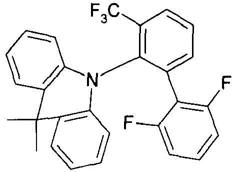 | 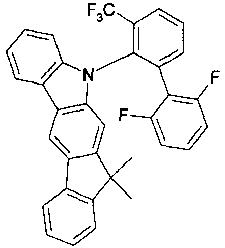 |
| 10 | 11 | 12 |
| 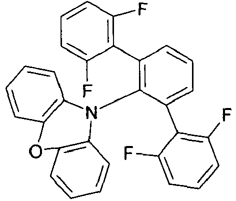 | 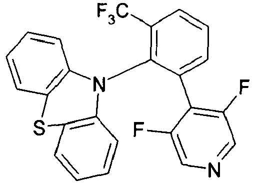 | 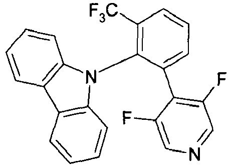 |
| 13 | 14 | 15 |
| | 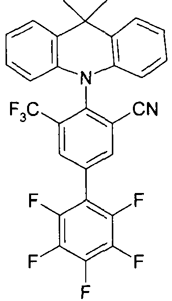 | 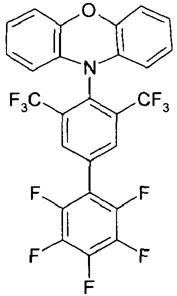 |
| | 17 | 18 |
| 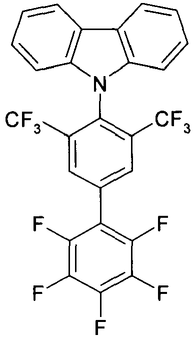 | 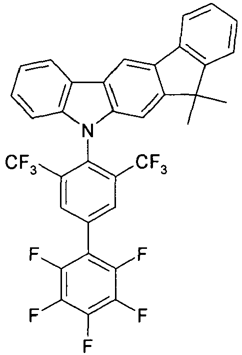 | 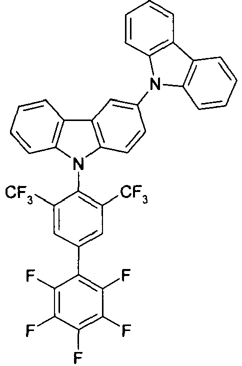 |
| 19 | 20 | 21 |
| 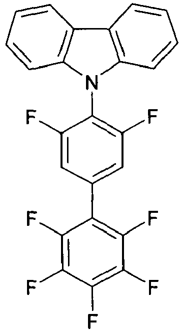 | 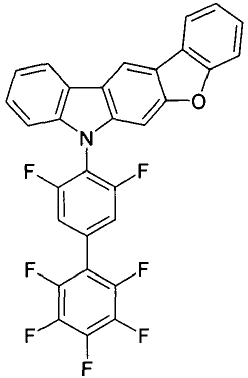 | 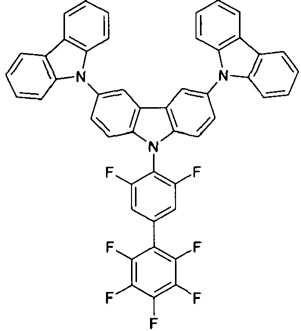 |
| 25 | 26 | 27 |
| 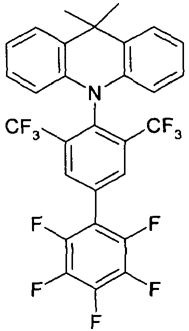 | 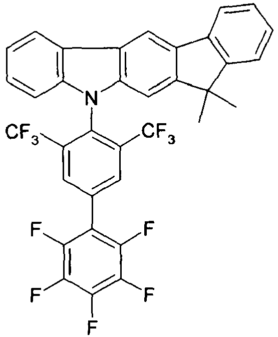 | 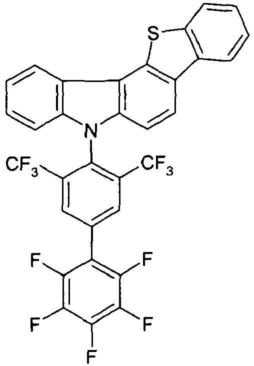 |
| 31 | 32 | 33 |
| 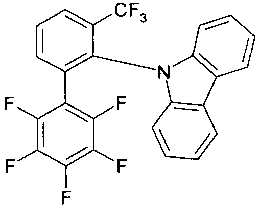 | 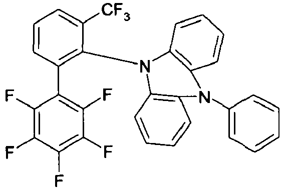 | 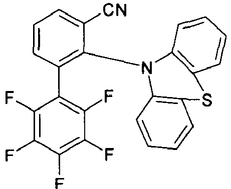 |
| 40 | 41 | 42 |
| 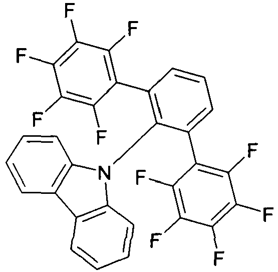 | 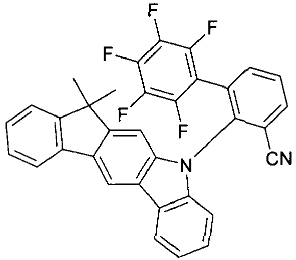 | 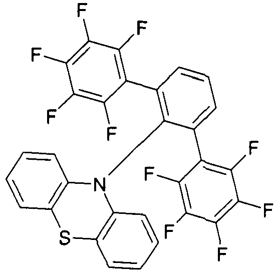 |
| 43 | 44 | 45 |
| 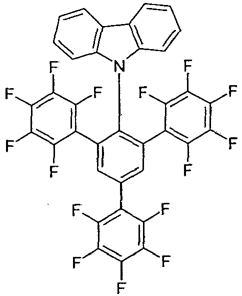 | 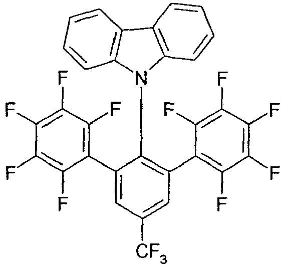 | 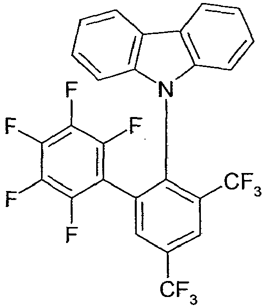 |
| 46 | 47 | 48 |
| 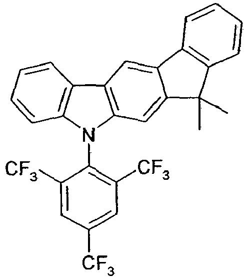 | 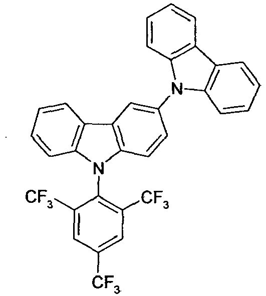 | 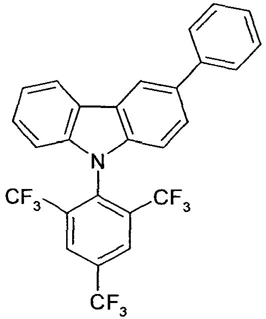 |
| 49 | 50 | 51 |
| 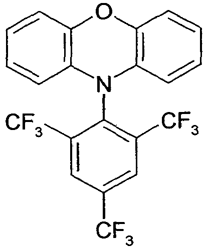 | 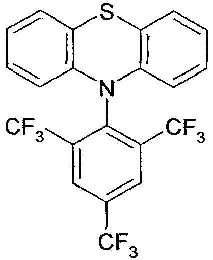 | 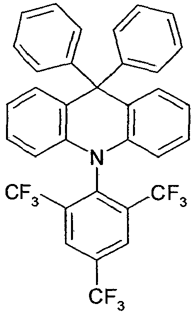 |
| 52 | 53 | 54 |
| 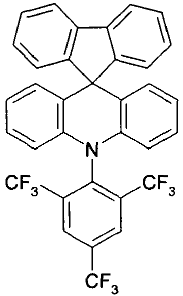 | 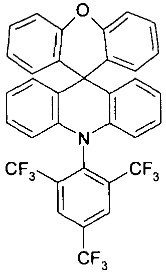 | 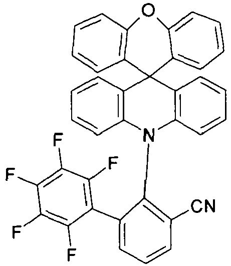 |
| 55 | 56 | 57 |
| 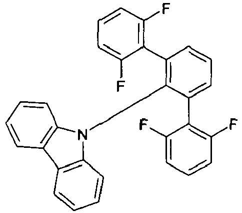 | 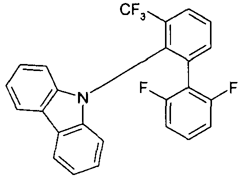 | 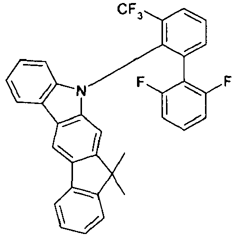 |
| 58 | 59 | 60 |
| 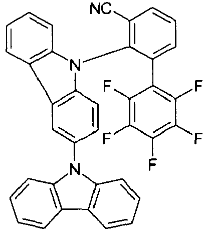 | 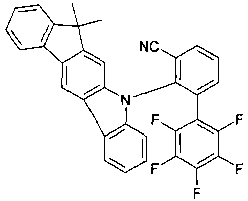 | 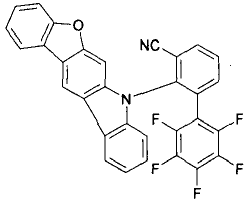 |
| 61 | 62 | 63 |
| | | 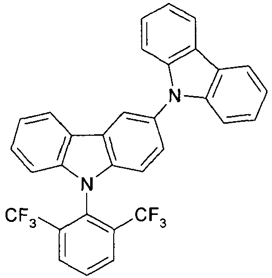 |
| | | 66 |
| 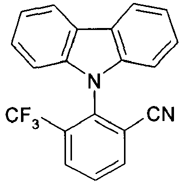 | 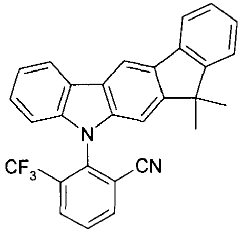 | 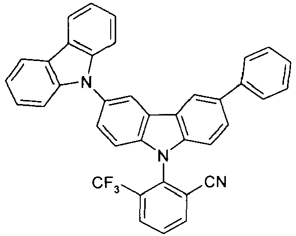 |
| 67 | 68 | 69 |
| 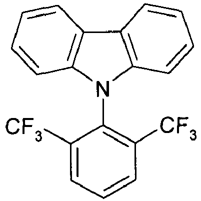 | 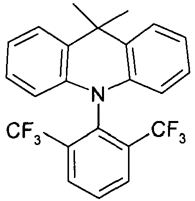 | 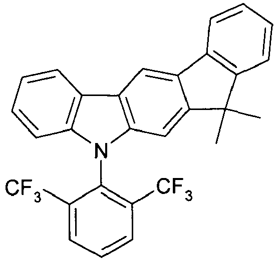 |
| 70 | 71 | 72 |
| 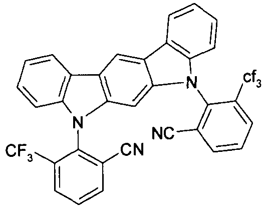 | 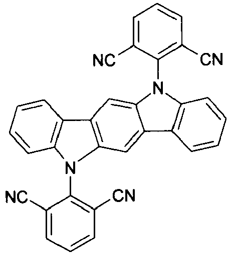 | |
| 76 | 77 | |
| | 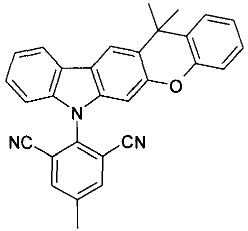 | 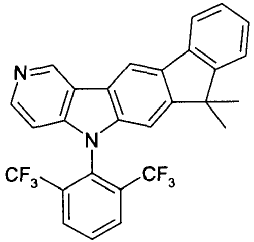 |
| | 80 | 81 |
| | | 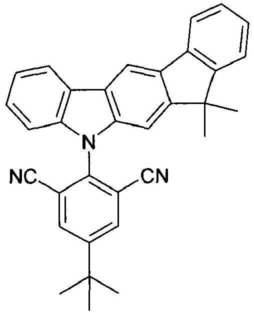 |
| | | 84 |
| 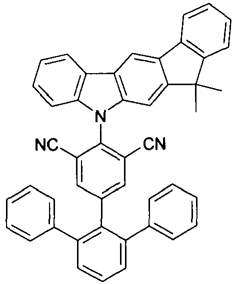 | 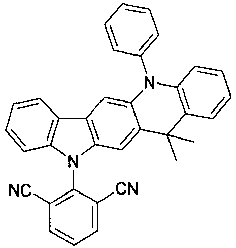 | |
| 85 | 86 | |
| | 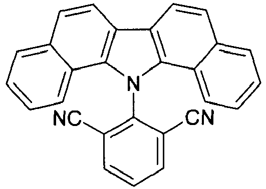 | 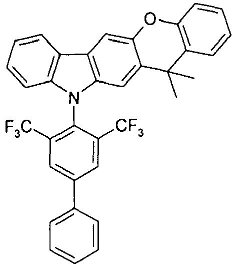 |
| | 89 | 90 |
| 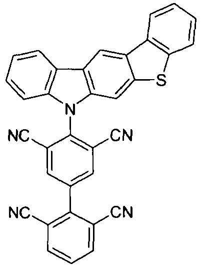 | | |
| 94 | | |
| | 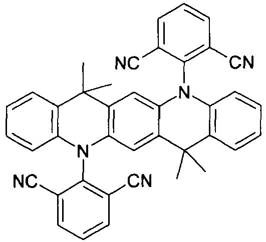 | |
| | 98 | |
| 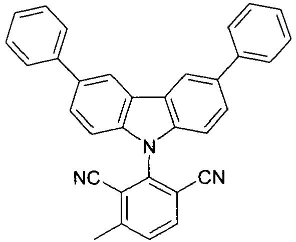 | 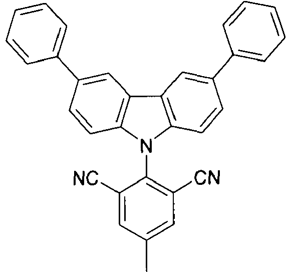 | 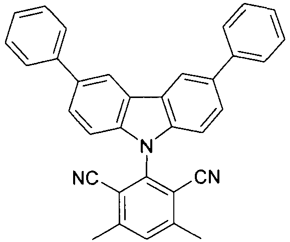 |
| 100 | 101 | 102 |
| 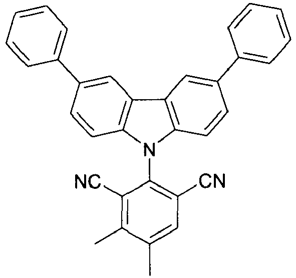 | 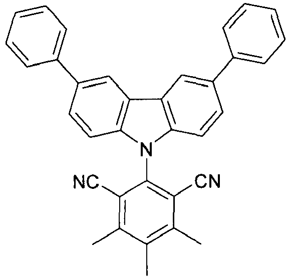 | 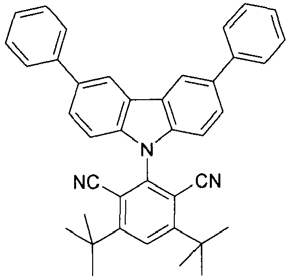 |
| 103 | 104 | 105 |
| 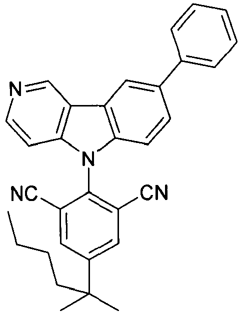 | 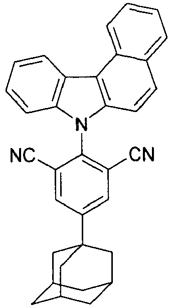 | |
| 106 | 107 | |
| 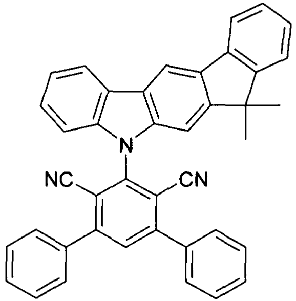 | 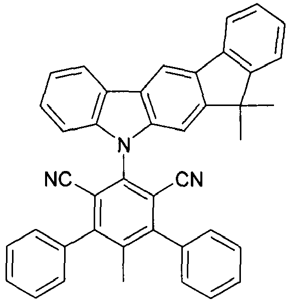 | 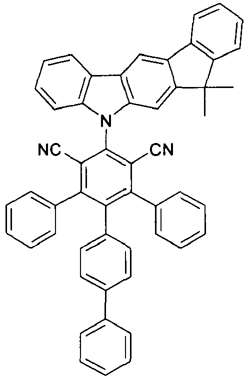 |
| 109 | 110 | 111 |
| 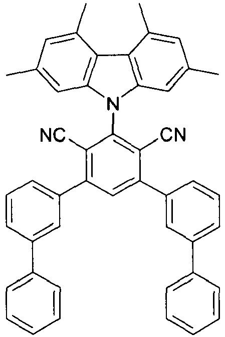 | 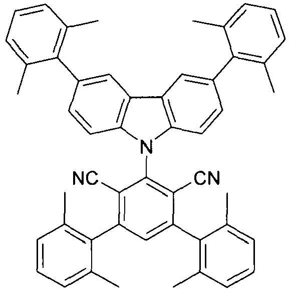 | 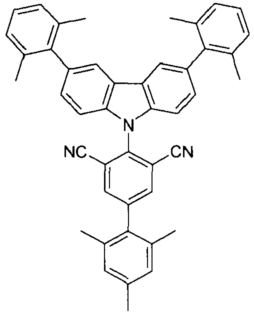 |
| 112 | 113 | 114 |
| 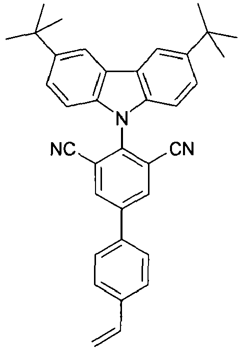 | 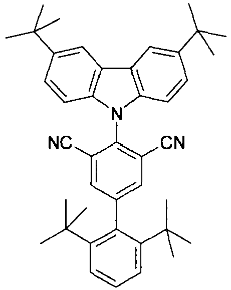 | |
| 115 | 116 | |
| 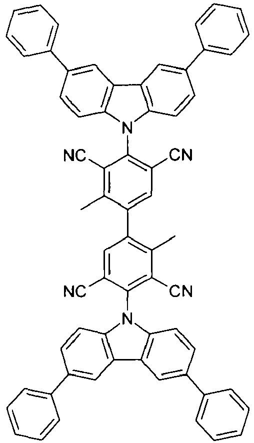 | 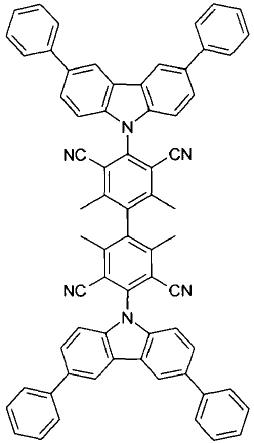 | |
| 118 | 119 | |
| | | 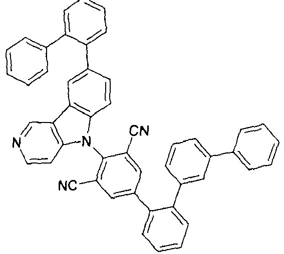 |
| | | 163 |
| | | 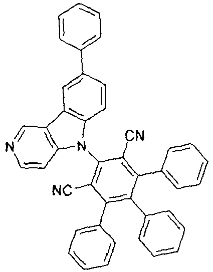 |
| | | 166 |
| 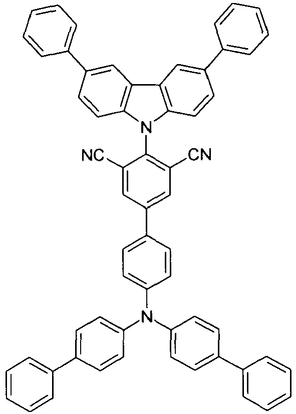 | 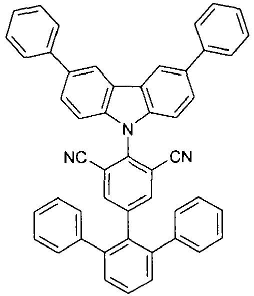 | |
| 185 | 186 | |

Die Verbindungen gemäß Formel (I) können unter Verwendung von bekannten organisch-chemischen Syntheseschritten hergestellt werden, beispielsweise Bromierung, Boronierung, nukleophile aromatische Substitution und übergangsmetallkatalysierte Kupplungsreaktion, beispielsweise Buchwald-Kupplung.

w Schema 1 zeigt ein beispielhaftes Verfahren zur Herstellung von Verbindungen gemäß Formel (I), bei dem von einer Cyano-substituierten Verbindung (in vielen Fällen kommerziell erhältlich), die einen oder mehrere Fluor-Substituenten trägt, ausgegangen wird. Dabei werden durch nukleophile aromatische Substitution eine oder mehrere Carbazolderivate eingeführt. Das Verfahren kann schrittweise erfolgen, so dass zunächst ein Carbazolderivat, und dann anschließend ein weiteres, anderes Carbazolderivat, eingeführt werden kann (s. untere Zeile). Anstelle von CN können andere elektronenziehende Gruppen eingesetzt werden. Die gezeigten Verbindungen können beliebig weiter substitutiert sein, gemäß den in Anspruch 1 definierten Substituenten. Dies gilt für alle folgenden Schemata, die Syntheseverfahren erläutern.

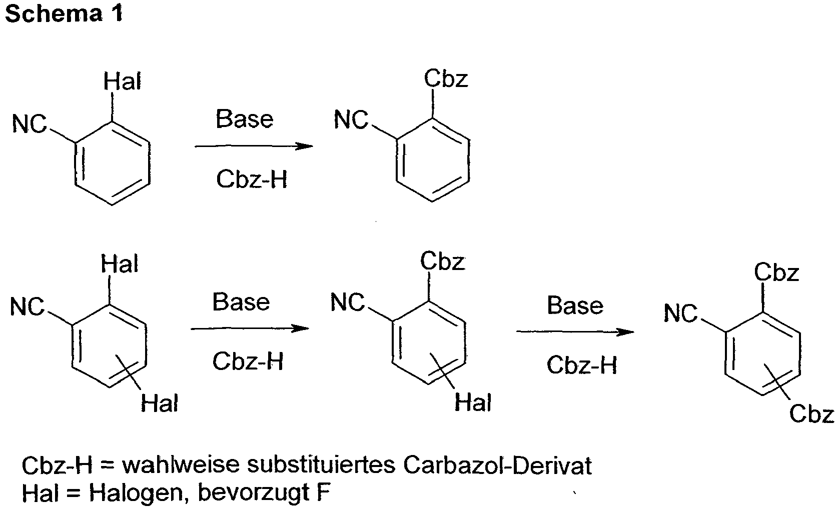

Weiterhin können Carbazolderivate auch durch Buchwald-Kupplung in die erfindungsgemäßen Verbindungen eingeführt werden. Dies ist beispielhaft in Schema 2 gezeigt für eine Verbindung, die eine CF₃-Gruppe trägt. Es könnten jedoch auch andere Gruppen, wie CN oder F oder elektronenarme Heteroaromaten, statt CF₃ vorhanden sein.

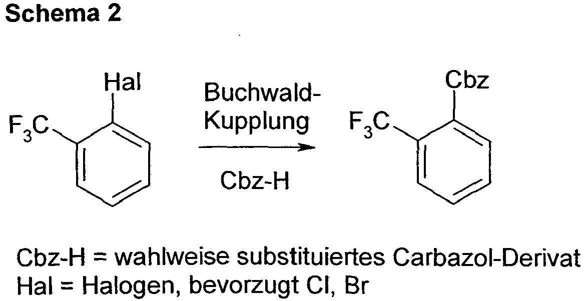

Ein zu Schema 2 alternatives Verfahren zur Einführung eines Carbazolderivats zeigt Schema 3. Dabei wird von einem Nitro-Benzolderivat ausgegangen, das zu einem Amino-Benzolderivat umgesetzt wird, welches dann mit einem geeigneten halogensubstituierten Biaryl eine doppelte Buchwald-Kupplung eingeht. Anstelle der gezeigten C₆F₅-Gruppe können auch andere Gruppen, beispielsweise F, CF₃ oder CN, vorhanden sein.

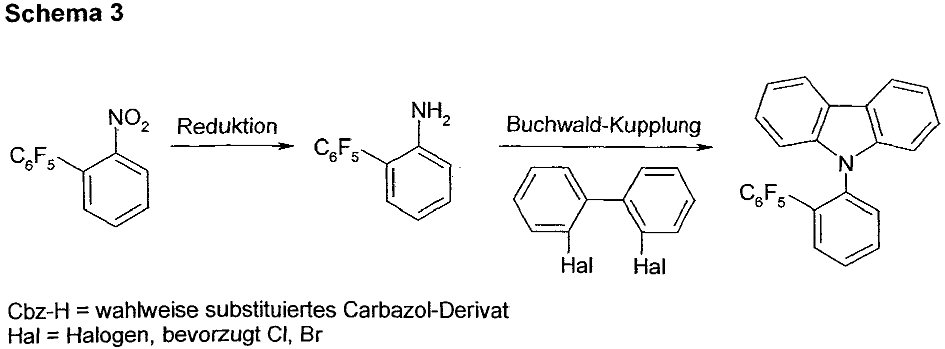

Verbindungen, in denen ein Carbazolderivat in benachbarter Position zu einem elektronenziehenden Heteroaromaten vorliegt, können wie im folgenden Schema 4 gezeigt hergestellt werden. Hierzu wird von einem Carbazol-Phenyl-Derivat ausgegangen, welches einen Halogensubstituenten oder eine andere geeignete Abgangsgruppe aufweist. Diese Verbindung wird in eine Boronsäure überführt, mit der dann in einem zweiten Schritt eine Suzuki-Kupplung mit einem elektronenarmen Aromaten durchgeführt wird. Durch zweifache Suzuki-Kupplung können Verbindungen hergestellt werden, die zwei unterschiedliche Heteroaryl-Substituenten tragen.

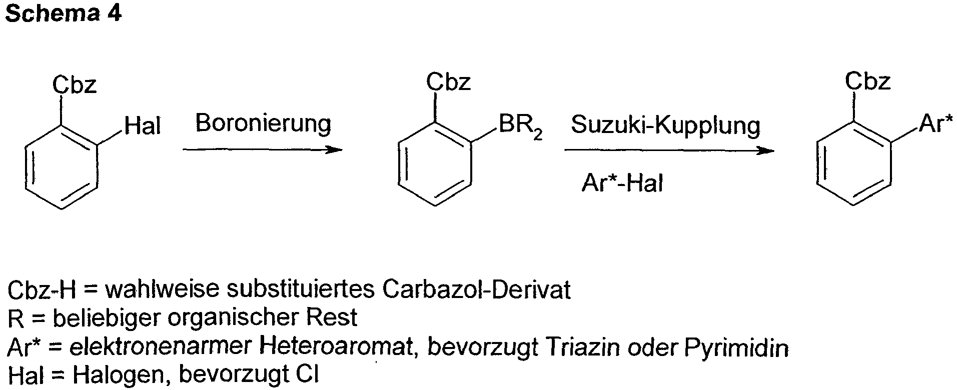

Die oben gezeigten Syntheseverfahren sind Anhaltspunkte für den Fachmann, wie erfindungsgemäße Verbindungen über die gesamte Breite der Ansprüche der vorliegenden Anmeldung hergestellt werden können. Der Fachmann wird zur näheren Erläuterung die konkreten Ausführungsbeispiele heranziehen, die in der Anmeldung enthalten sind. Er zieht weiterhin sein allgemeines Fachwissen über organisch-chemische Syntheseverfahren heran und bedient sich kommerziell erhältlicher Verbindungen als Ausgangsstoffe, um erfindungsgemäße Verbindungen herzustellen, deren Synthese vorliegend nicht explizit beschrieben ist. Weiterhin kann er im Rahmen seines allgemeinen Fachwissens die hier beschriebenen Verfahren abwandeln, wenn dies praktische Vorteile mit sich bringt.

Gegenstand der vorliegenden Erfindung ist zusammenfassend ein Verfahren zur Herstellung einer Verbindung gemäß Formel (I), dadurch gekennzeichnet, dass mindestens ein Carbazolderivat durch nukleophile aromatische Substitution oder Buchwald-Kupplung eingeführt wird, oder dass mindestens eine elektronenarme Heteroarylgruppe durch Suzuki-Kupplung eingeführt wird. Bevorzugt sind die oben angegebenen konkreten Syntheseverfahren.

Die erfindungsgemäßen Verbindungen, insbesondere Verbindungen, welche mit reaktiven Abgangsgruppen, wie Brom, Iod, Chlor, Boronsäure oder Boronsäureester, substituiert sind, können als Monomere zur Erzeugung entsprechender Oligomere Verwendung finden. Geeignete reaktive Abgangsgruppen sind beispielsweise Brom, Iod, Chlor, Boronsäuren, Boronsäureester, Amine, Alkenyl- oder Alkinylgruppen mit endständiger C-C-Doppelbindung bzw. C-C-Dreifachbindung, Oxirane, Oxetane, Gruppen, die eine Cycloaddition, beispielsweise eine 1,3-dipolare Cycloaddition, eingehen, wie beispielsweise Diene oder Azide, Carbonsäurederivate, Alkohole und Silane.

Weiterer Gegenstand der Erfindung sind daher Oligomere, enthaltend eine oder mehrere Verbindungen gemäß Formel (I), wobei die Bindung(en) zum Oligomer an beliebigen, in Formel (I) mit R¹ oder R² substituierten Positionen lokalisiert sein können. Je nach Verknüpfung der Verbindung gemäß Formel (I) ist die Verbindung Bestandteil einer Seitenkette des Oligomers oder Bestandteil der Hauptkette. Unter einem Oligomer im Sinne dieser Erfindung wird eine Verbindung verstanden, welche aus mindestens vier Monomereinheiten und höchstens neun Monomereinheiten aufgebaut ist. Die erfindungsgemäßen Oligomere können konjugiert, teilkonjugiert oder nicht-konjugiert sein. Die erfindungsgemäßen Oligomere können linear oder verzweigt sein. In den linear verknüpften Strukturen können die Einheiten gemäß Formel (I) direkt miteinander verknüpft sein oder sie können über eine bivalente Gruppe, beispielsweise über eine substituierte oder unsubstituierte Alkylengruppe, über ein Heteroatom oder über eine bivalente aromatische oder heteroaromatische Gruppe miteinander verknüpft sein. In verzweigten Strukturen können beispielsweise drei oder mehrere Einheiten gemäß Formel (I) über eine trivalente oder höhervalente Gruppe, beispielsweise über eine trivalente oder höhervalente aromatische oder heteroaromatische Gruppe, zu einem verzweigten Oligomer verknüpft sein.

Für die Wiederholeinheiten gemäß Formel (I) in Oligomeren gelten dieselben Bevorzugungen wie oben für Verbindungen gemäß Formel (I) beschrieben.

Die erfindungsgemäßen Oligomere können durch Oligomerisation von einer oder mehreren Monomersorten hergestellt werden, von denen mindestens ein Monomer im Oligomer zu Wiederholungseinheiten der Formel (I) führt. Zur Herstellung der Oligomere werden die erfindungsgemäßen Monomere beispielsweise homopolymerisiert oder mit weiteren Monomeren copolymerisiert. Geeignete Oligomerisationsreaktionen sind dem Fachmann bekannt und in der Literatur beschrieben.

Die Oligomere können jedoch auch durch schrittweise organische Synthese hergestellt werden.

Die erfindungsgemäßen Oligomere weisen vorteilhafte Eigenschaften, insbesondere hohe Lebensdauern, hohe Effizienzen und gute Farbkoordinaten auf.

Die Verbindung gemäß Formel (I) eignet sich für den Einsatz in einer elektronischen Vorrichtung, insbesondere einer organischen Elektrolumineszenzvorrichtung (OLED). Abhängig von der Substitution kann die Verbindung der Formel (I) in unterschiedlichen Funktionen und Schichten eingesetzt werden. Bevorzugt ist die Verwendung in einer emittierenden Schicht, besonders bevorzugt als emittierende Verbindung in einer emittierenden Schicht.

Weiterer Gegenstand der Erfindung ist daher die Verwendung einer Verbindung gemäß Formel (I) in einer elektronischen Vorrichtung. Dabei ist die elektronische Vorrichtung bevorzugt ausgewählt aus der Gruppe bestehend aus organischen integrierten Schaltungen (OICs), organischen Feld-Effekt-Transistoren (OFETs), organischen Dünnfilmtransistoren (OTFTs), organischen lichtemittierenden Transistoren (OLETs), organischen Solarzellen (OSCs), organischen optischen Detektoren, organischen Photorezeptoren, organischen Feld-Quench-Devices (OFQDs), organischen lichtemittierenden elektrochemischen Zellen (OLECs), organischen Laserdioden (O-Laser) und besonders bevorzugt organischen Elektrolumineszenzvorrichtungen (OLEDs).

Gegenstand der Erfindung ist weiterhin eine elektronische Vorrichtung, enthaltend mindestens eine Verbindung gemäß Formel (I). Dabei ist die elektronische Vorrichtung bevorzugt ausgewählt aus den oben genannten Vorrichtungen.

Besonders bevorzugt ist eine organische Elektrolumineszenzvorrichtung, enthaltend Anode, Kathode und mindestens eine emittierende Schicht, dadurch gekennzeichnet, dass mindestens eine organische Schicht in der Vorrichtung enthalten ist, welche mindestens eine Verbindung gemäß Formel (I) enthält. Bevorzugt ist eine organische Elektrolumineszenzvorrichtung, enthaltend Anode, Kathode und mindestens eine emittierende Schicht, dadurch gekennzeichnet, dass mindestens eine organische Schicht in der Vorrichtung, gewählt aus emittierenden Schichten, mindestens eine Verbindung gemäß Formel (I) enthält.

Außer Kathode, Anode und emittierender Schicht kann die elektronische Vorrichtung noch weitere Schichten enthalten. Diese sind beispielsweise gewählt aus jeweils einer oder mehreren Lochinjektionsschichten, Lochtransportschichten, Lochblockierschichten, Elektronentransportschichten, Elektroneninjektionsschichten, Elektronenblockierschichten, Excitonenblockierschichten, Zwischenschichten (Interlayers), Ladungserzeugungsschichten (Charge-Generation Layers) (IDMC 2003, Taiwan; Session 21 OLED (5), T. Matsumoto, T. Nakada, J. Endo, K. Mori, N. Kawamura, A. Yokoi, J. Kido, Multiphoton Organic EL Device Having Charge Generation Layer) und/oder organischen oder anorganischen p/n-Übergängen. Es sei aber darauf hingewiesen, dass nicht notwendigerweise jede dieser Schichten vorhanden sein muss und die Wahl der Schichten immer von den verwendeten Verbindungen abhängt und insbesondere auch von der Tatsache, ob es sich um eine fluoreszierende oder phosphoreszierende Elektrolumineszenzvorrichtung handelt.

Die Abfolge der Schichten der elektronischen Vorrichtung ist bevorzugt wie folgt:
- Anode-
- Lochinjektionsschicht-
- Lochtransportschicht-
- optional weitere Lochtransportschichten-
- emittierende Schicht-
- Elektronentransportschicht-
- Elektroneninjektionsschicht-
- Kathode-.
Es müssen dabei nicht alle der genannten Schichten vorhanden sein, und es können zusätzlich weitere Schichten vorhanden sein.

Dem Fachmann sind aus der Fachliteratur geeignete Verbindungen bekannt, die er in den entsprechenden Schichten einsetzen kann.

Als Kathode der elektronischen Vorrichtung sind Metalle mit geringer Austrittsarbeit, Metalllegierungen oder mehrlagige Strukturen aus verschiedenen Metallen bevorzugt, wie beispielsweise Erdalkalimetalle, Alkalimetalle, Hauptgruppenmetalle oder Lanthanoide (z. B. Ca, Ba, Mg, Al, In, Mg, Yb, Sm, etc.). Weiterhin eignen sich Legierungen aus einem Alkali- oder Erdalkalimetall und Silber, beispielsweise eine Legierung aus Magnesium und Silber. Bei mehrlagigen Strukturen können auch zusätzlich zu den genannten Metallen weitere Metalle verwendet werden, die eine relativ hohe Austrittsarbeit aufweisen, wie z. B. Ag oder Al, wobei dann in der Regel Kombinationen der Metalle, wie beispielsweise Ca/Ag, Mg/Ag oder Ba/Ag verwendet werden. Es kann auch bevorzugt sein, zwischen einer metallischen Kathode und dem organischen Halbleiter eine dünne Zwischenschicht eines Materials mit einer hohen Dielektrizitätskonstante einzubringen. Hierfür kommen beispielsweise Alkalimetall- oder Erdalkalimetallfluoride, aber auch die entsprechenden Oxide oder Carbonate in Frage (z. B. LiF, Li₂O, BaF₂, MgO, NaF, CsF, Cs₂CO₃, etc.). Weiterhin kann dafür Lithiumchinolinat (LiQ) verwendet werden.

Als Anode sind Materialien mit hoher Austrittsarbeit bevorzugt. Bevorzugt weist die Anode eine Austrittsarbeit größer 4.5 eV vs. Vakuum auf. Hierfür sind einerseits Metalle mit hohem Redoxpotential geeignet, wie beispielsweise Ag, Pt oder Au. Es können andererseits auch Metall/Metalloxid-Elektroden (z. B. Al/Ni/NiOₓ, Al/PtOₓ) bevorzugt sein. Für einige Anwendungen muss mindestens eine der Elektroden transparent oder teiltransparent sein, um entweder die Bestrahlung des organischen Materials (organische Solarzelle) oder die Auskopplung von Licht (OLED, O-LASER) zu ermöglichen. Bevorzugte Anodenmaterialien sind hier leitfähige gemischte Metalloxide. Besonders bevorzugt sind Indium-ZinnOxid (ITO) oder Indium-Zink Oxid (IZO). Bevorzugt sind weiterhin leitfähige, dotierte organische Materialien, insbesondere leitfähige dotierte Polymere. Weiterhin kann die Anode auch aus mehreren Schichten bestehen, beispielsweise aus einer inneren Schicht aus ITO und einer äußeren Schicht aus einem Metalloxid, bevorzugt Wolframoxid, Molybdänoxid oder Vanadiumoxid.

Wenn die Verbindung gemäß Formel (I) als emittierende Verbindung in einer emittierenden Schicht verwendet wird, ist es bevorzugt, dass als weitere Komponente in der Schicht eine Matrixverbindung vorhanden ist, wie oben definiert. Dabei können als Matrixverbindungen die dem Fachmann hierzu bekannten Verbindungen verwendet werden, beispielsweise 4,4'-(Biscarbazol-9-yl)-biphenyl (CBP), oder 2,8-Bis(diphenylphosphoryl)dibenzo[b,d]thiophen (PPT).

Weiterhin ist es im Fall der Verwendung als emittierende Verbindung bevorzugt, dass die Verbindung in einem Anteil zwischen 0.1 und 50.0 Vol.-%, besonders bevorzugt zwischen 0.5 und 20.0 Vol.-% und ganz besonders bevorzugt zwischen 0.5 und 8.0 Vol.-% in der emittierenden Schicht vorliegt.

Zur Herstellung der elektronischen Vorrichtung sind dem Fachmann geeignete Verfahren bekannt. Insbesondere wird die Vorrichtung nach Auftragung der Schichten entsprechend (je nach Anwendung) strukturiert, kontaktiert und schließlich versiegelt, um schädigende Effekte von Wasser und Luft auszuschließen.

Elektronische Vorrichtungen enthaltend eine oder mehrere Verbindungen gemäß Formel (I) können unter anderem in Displays, als Lichtquellen in Beleuchtungsanwendungen sowie als Lichtquellen in medizinischen oder kosmetischen Anwendungen (z.B. Lichttherapie) eingesetzt werden.

### Ausführungsbeispiele (nicht anspruchsgemäße Beispiele sind mit# gekennzeichnet)

### A) Synthesebeispiele:

Die nachfolgenden Synthesen werden, sofern nicht anders angegeben, unter einer Schutzgasatmosphäre in getrockneten Lösungsmitteln durchgeführt. Die Metallkomplexe werden zusätzlich unter Ausschluss von Licht bzw. unter Gelblicht gehandhabt. Die Lösungsmittel und Reagenzien können z.B. von Sigma-ALDRICH bzw. ABCR bezogen werden.

### I) Synthese von erfindungsgemäßen Verbindungen: Beispiel S1: 1,3,5-Tris-cyano-2,4,6-tris(N-carbazolyl)benzol #

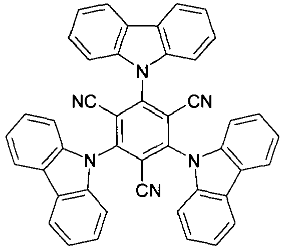

### Variante A:

Eine gut gerührte Suspension von 16.0 g (400 mmol) Natriumhydrid, 60 Gew.-% ige Dispersion in Mineralöl, in 500 ml THF wird unter Eiskühlung bei ca. + 10 °C portionsweise mit 66.9 g (400 mmol) Carbazol [51555-21-6] versetzt - Vorsicht Wasserstoffentwicklung! Schäumen! Nach beendeter Zugabe wird die Mischung 30 min. nachgerührt und dann portionsweise unter Eiskühlung so mit 20.7 g (100 mmol) 1,3,5-Tricyano-2,4,6-trifluorbenzol [363897-9] versetzt, dass die Temperatur + 20 °C nicht übersteigt. Nach beendeter Zugabe rührt man 2 h bei + 10 °C nach, entfernt dann das Kältebad, lässt auf 20 - 25 °C erwärmen, rührt 2 h nach und erwärmt dann noch 12 h auf 40 °C. Nach Abkühlen auf Raumtemperatur wird die Reaktion durch Zutropfen von 30 ml MeOH beendet und die Reaktionsmischung im Vakuum fast bis zur Trockene eingeengt. Der Rückstand wird zweimal mit je 600 ml eines Gemischs aus 400 ml Methanol und 200 ml Wasser und dann einmal mit 500 ml Methanol heiß ausgerührt. Die Reinigung erfolgt durch dreimalige Umkristallisation □us Dioxan (ca. 5 ml/g) anschließend fünfmalige Umkristallisation aus DMF (ca. 2.5 ml/g) und zweimalige fraktionierte Sublimation (p ca. 1 x 10⁻⁵ mbar, T ca. 310 - 320 °C). Ausbeute: 23.6 g (36.3 mmol) 36 %. Reinheit: 99.9 % n. HPLC.

### Variante B:

Durchführung analog zu Variante A, jedoch wird das Carbazol im THF vorgelegt und dann tropfenweise mit 160 ml (400 mmol) n-BuLi, 2.5 molar in n-Hexan versetzt.

Ausbeute: 19.0 g (29.3 mmol) 29 %. Reinheit: 99.9 % n. HPLC.

### Variante C:

Eine gut gerührte Suspension von 66.9 g (400 mmol) Carbazol [51555-21-6], 20.7 g (100 mmol) 1,3,5-Tricyano-2,4,6-trifluorbenzol, 106.1 g (500 mmol) Trikaliumphosphat (wasserfrei) und 200 g Glaskugeln wird in 500 ml Dimethylacetamid 16 h bei 160 °C gerührt. Nach Erkalten gibt man 1000 ml Wasser zu, saugt vom ausgefallenen Feststoff ab, wäscht diesen zweimal mit je 300 ml Wasser, zweimal mit je 200 ml Methanol und trocknet dann im Vakuum. Weitere Reinigung analog Variante A. Ausbeute: 20.5 g (31.6 mmol) 31 %. Reinheit: 99.9 % n. HPLC.

Analog können folgende Verbindungen dargestellt werden:

| Bsp. | Variante Edukte | Produkt | Ausbeute |
|---|---|---|---|
| S2 # | A | 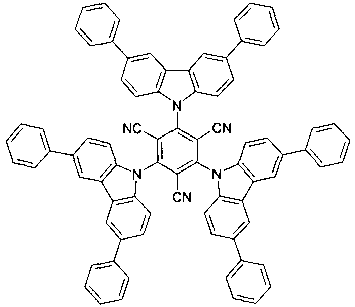 | 30 % |
| | 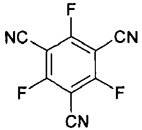 | | |
| | 363897-9 | | |
| | 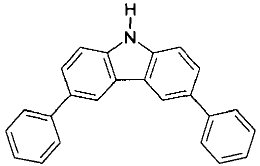 | | |
| | 56525-79-2 | | |
| S3 # | A | 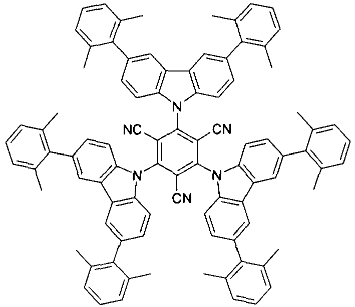 | 33 % |
| | 363897-9 | | |
| | 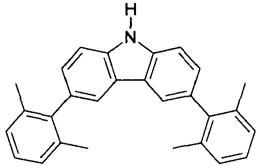 | | |
| | 1246891 | | |
| S4 # | A | 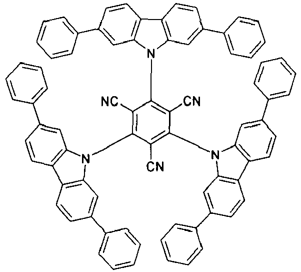 | 36 % |
| | 363897-9 | | |
| | 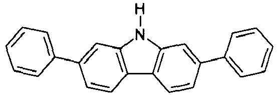 | | |
| | 42448-04-4 | | |
| S5 # | A | 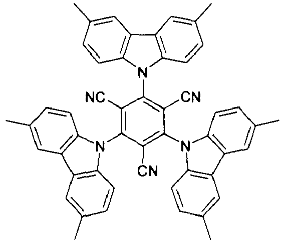 | 32 % |
| | 363897-9 | | |
| | 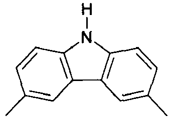 | | |
| | 5599-50-8 | | |
| S6 # | B | 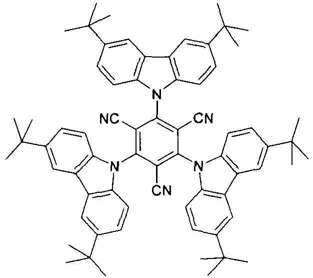 | 37 % |
| | 363897-9 | | |
| | 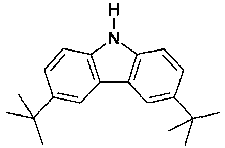 | | |
| | 37500-95-1 | | |
| S7 # | A | 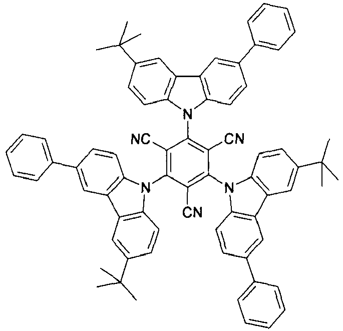 | 28 % |
| | 363897-9 | | |
| | 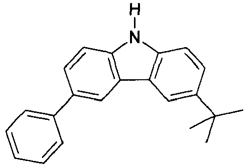 | | |
| | 1335126-51-6 | | |
| S8 # | A | 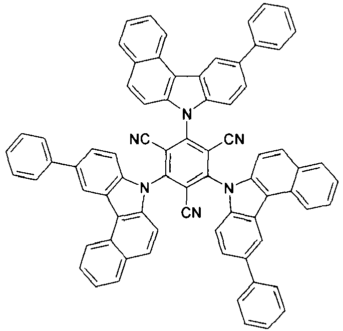 | 29 % |
| | 363897-9 | | |
| | 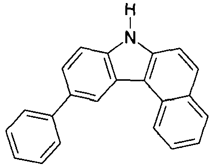 | | |
| | 756822-84-1 | | |
| S9 # | A | 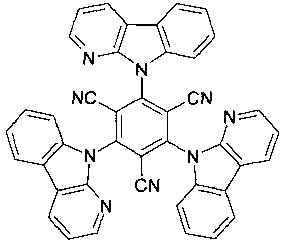 | 34 % |
| | 363897-9 | | |
| | 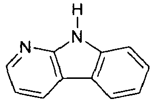 | | |
| | 244-76-8 | | |
| S10 # | C | 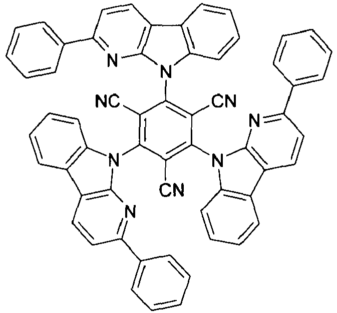 | 28 % |
| | 363897-9 | | |
| | 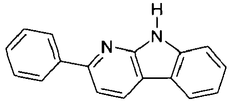 | | |
| | 64677-58-3 | | |
| S11 # | A | 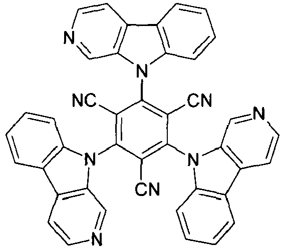 | 30 % |
| | 363897-9 | | |
| | 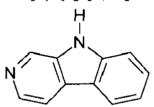 | | |
| | 244-63-3 | | |
| S12 # | A | 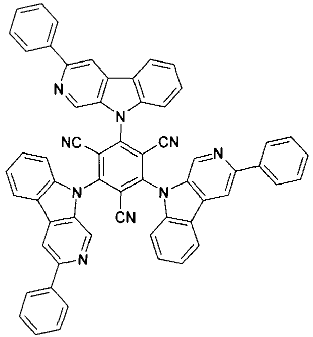 | 31 % |
| | 363897-9 | | |
| | 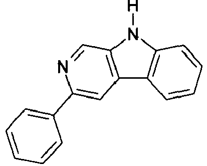 | | |
| | 91944-01-3 | | |
| S13 # | A | 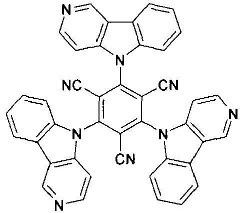 | 26 % |
| | 363897-9 | | |
| | 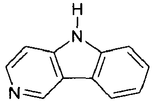 | | |
| | 244-69-9 | | |
| S14 # | A | 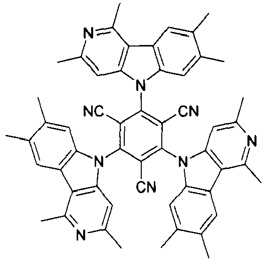 | 35 % |
| | 363897-9 | | |
| | 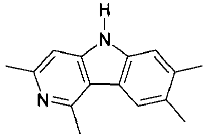 | | |
| | 180520-52-9 | | |
| S15 # | A | 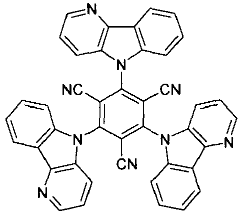 | 34 % |
| | 363897-9 | | |
| | 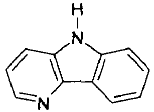 | | |
| | 245-08-9 | | |
| S16 # | A | 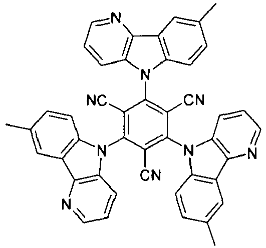 | 32 % |
| | 363897-9 | | |
| | 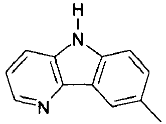 | | |
| | 1041015-29-5 | | |
| S17 # | A | 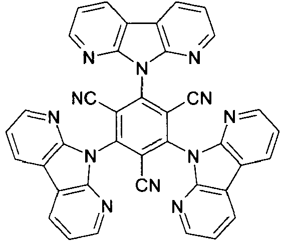 | 23 % |
| | 363897-9 | | |
| | 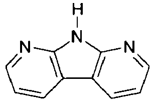 | | |
| | 17966-00-6 | | |
| S18 # | A | 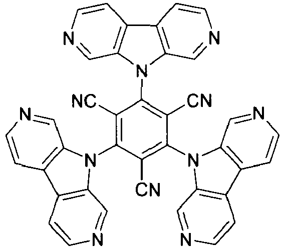 | 25 % |
| | 363897-9 | | |
| | 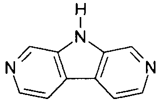 | | |
| | 77200-36-3 | | |
| S19 # | A | 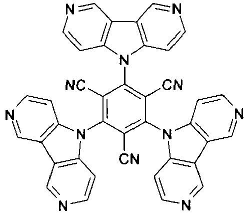 | 26 % |
| | 363897-9 | | |
| | 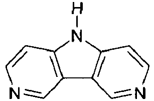 | | |
| | 244-78-0 | | |
| S20 # | C | 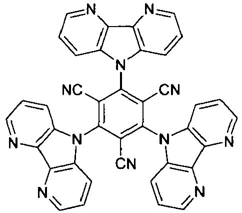 | 30 % |
| | 363897-9 | | |
| | 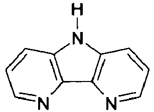 | | |
| | 75449-34-2 | | |
| S21 # | A | 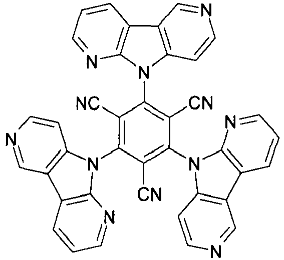 | 21 % |
| | 363897-9 | | |
| | 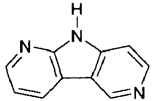 | | |
| | 26422-85-5 | | |
| S22 # | A | 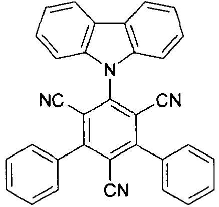 | 45 % |
| | 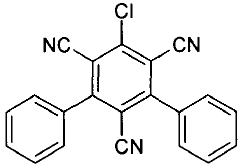 | | |
| | 274258-60-5 | Es werden - auch im Folgenden - 200 mmol Carbazol-Baustein eingesetzt. | |
| | 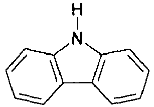 | | |
| | 51555-21-6 | Es wird 16 h bei 60 °C nachgerührt. | |
| S23 # | A | 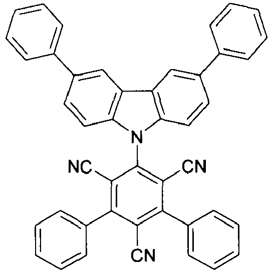 | 40 % |
| | 274258-60-5 | | |
| | 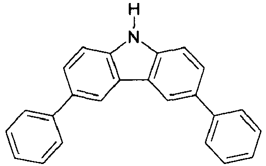 | | |
| | 56525-79-2 | | |
| S24 # | A | 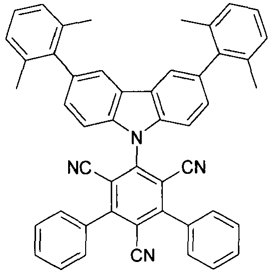 | 41 % |
| | 274258-60-5 | | |
| | 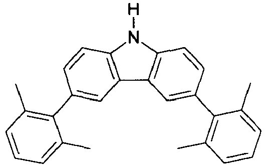 | | |
| | 1246891 | | |
| S25 # | A | 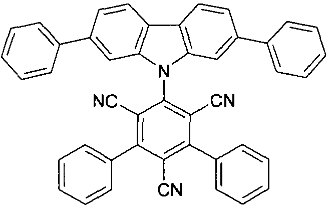 | 38 % |
| | 274258-60-5 | | |
| | 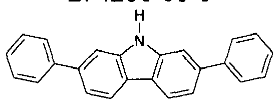 | | |
| | 42448-04-4 | | |
| S26 # | B | 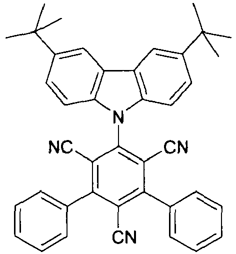 | 39 % |
| | 274258-60-5 | | |
| | 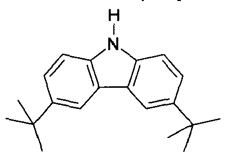 | | |
| | 37500-95-1 | | |
| S27 # | A | 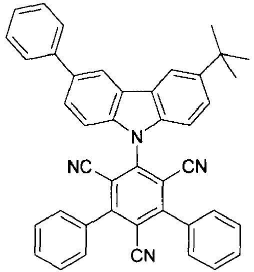 | 34 % |
| | 274258-60-5 | | |
| | 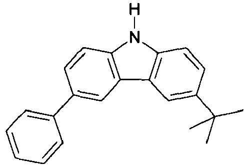 | | |
| | 1335126-51-6 | | |
| S28 # | A | 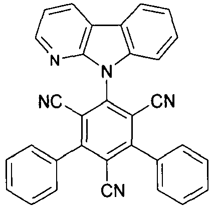 | 35 % |
| | 274258-60-5 | | |
| | 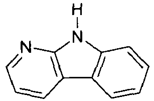 | | |
| | 244-76-8 | | |
| S29 # | B | 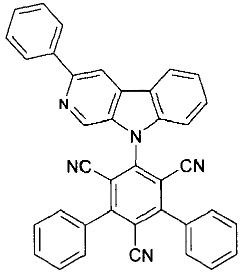 | 33 % |
| | 274258-60-5 | | |
| | 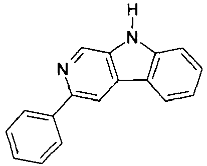 | | |
| | 91944-01-3 | | |
| S30 # | A | 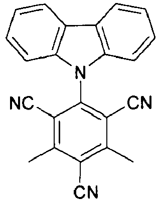 | 16% |
| | 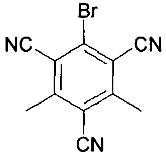 | | |
| | 80717-48-2 | Es werden - auch im Folgenden - 200 mmol Carbazol-Baustein eingesetzt. | |
| | 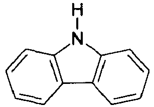 | Es wird 16 h bei 60 °C nachgerührt. | |
| | 51555-21-6 | | |
| S31 # | A | 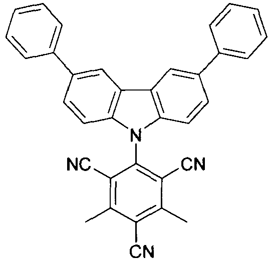 | 15% |
| | 80717-48-2 | | |
| | 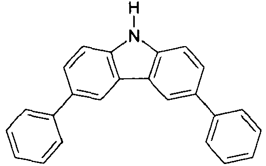 | | |
| | 56525-79-2 | | |
| S32 # | B | 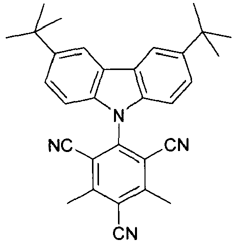 | 17% |
| | 80717-48-2 | | |
| | 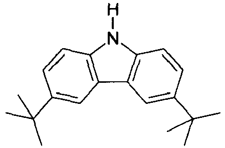 | | |
| | 37500-95-1 | | |
| S33 # | C | 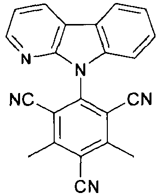 | 13 % |
| | 80717-48-2 | | |
| | 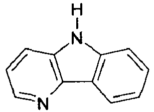 | | |
| | 245-08-9 | | |
| S34 # | 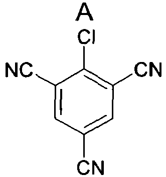 | 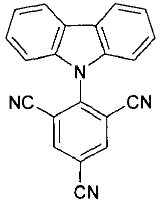 | 36 % |
| | 34760-78-8 | Es werden - auch im Folgenden - 200 mmol Carbazol-Baustein eingesetzt. | |
| | 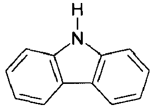 | | |
| | 51555-21-6 | Es wird 16 h bei 60 °C nachgerührt. | |
| S35 # | A | 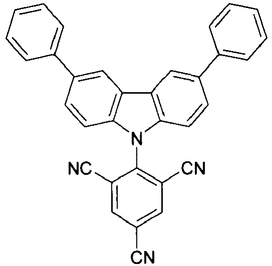 | 35 % |
| | 34760-78-8 | | |
| | 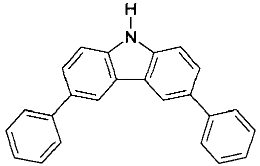 | | |
| | 56525-79-2 | | |
| S36 # | A | 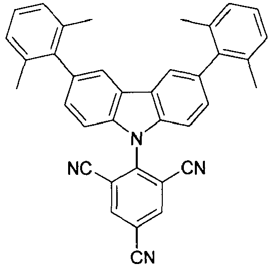 | 31 % |
| | 34760-78-8 | | |
| | 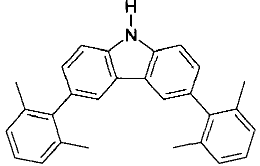 | | |
| | 1246891 | | |
| S37 # | A | 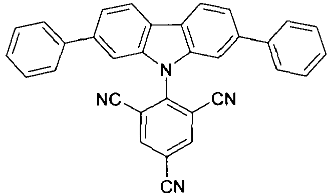 | 34 % |
| | 34760-78-8 | | |
| | 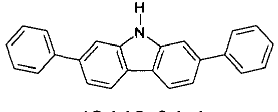 | | |
| | 42448-04-4 | | |
| S38 # | A | 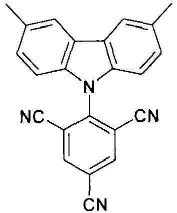 | 33 % |
| | 34760-78-8 | | |
| | 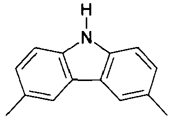 | | |
| | 5599-50-8 | | |
| S39 # | C | 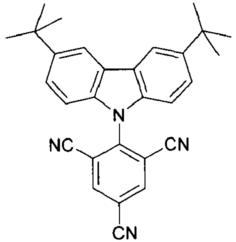 | 29 % |
| | 34760-78-8 | | |
| | 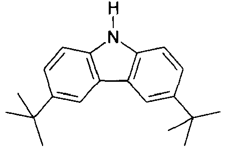 | | |
| | 37500-95-1 | | |
| S40 # | A | 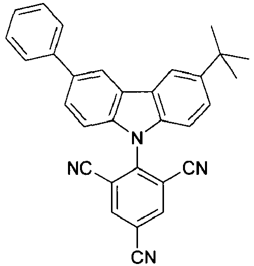 | 30% |
| | 34760-78-8 | | |
| | 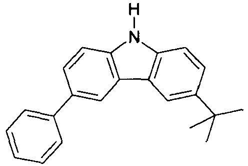 | | |
| | 1335126-51-6 | | |
| S41 # | A | 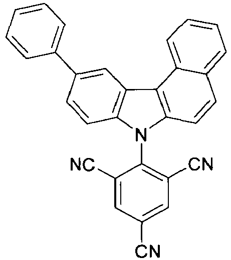 | 26 % |
| | 34760-78-8 | | |
| | 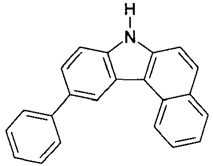 | | |
| | 756822-84-1 | | |
| S42 # | A | 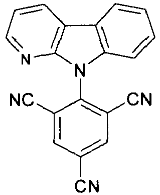 | 28 % |
| | 34760-78-8 | | |
| | 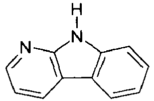 | | |
| | 244-76-8 | | |
| S43 # | A | 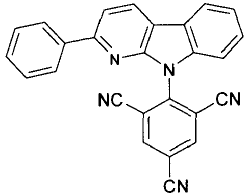 | 32 % |
| | 34760-78-8 | | |
| | 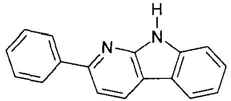 | | |
| | 64677-58-3 | | |
| S44 # | A | 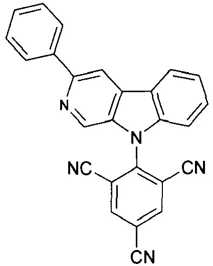 | 31% |
| | 34760-78-8 | | |
| | 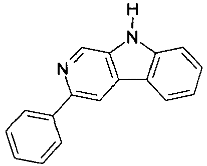 | | |
| | 91944-01-3 | | |
| S45 # | B | 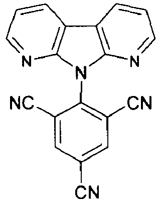 | 24% |
| | 34760-78-8 | | |
| | 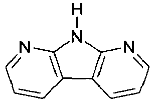 | | |
| | 17966-00-6 | | |
| S46 # | A | 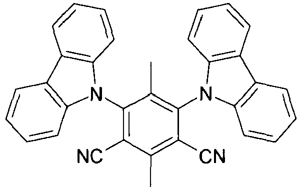 | 19 % |
| | 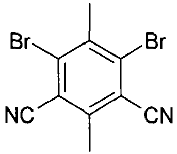 | | |
| | 499154-29-9 | | |
| | 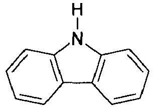 | Es werden - auch im Folgenden - 200 mmol Carbazol-Baustein eingesetzt. | |
| | 51555-21-6 | Es wird 16 h bei 60 °C nachgerührt. | |
| S47 # | A | 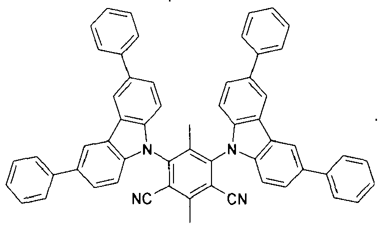 | 18 % |
| | 499154-29-9 | | |
| | 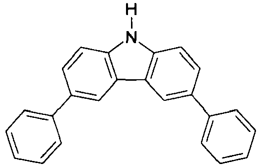 | | |
| | 56525-79-2 | | |
| S48 # | A | 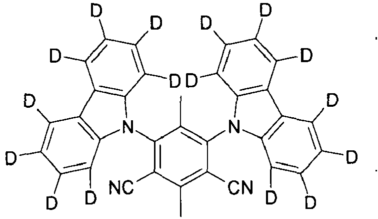 | 20 % |
| | 499154-29-9 | | |
| | 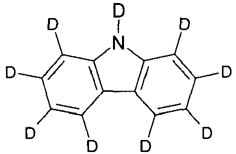 | | |
| | 97960-58-2 | | |
| S49 # | A | 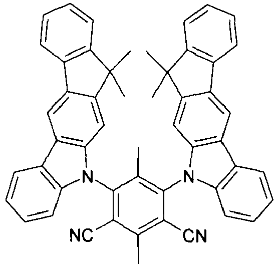 | 19 % |
| | 499154-29-9 | | |
| | 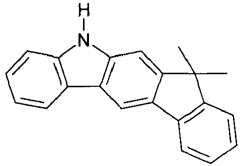 | | |
| | 1257220-47-5 | | |
| S50 | A | 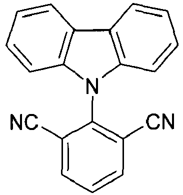 | 53 % |
| | 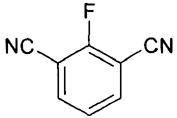 | | |
| | 23039-06-7 | | |
| | 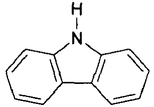 | Es werden - auch im Folgenden - 200 mmol Carbazol-Baustein eingesetzt | |
| | 51555-21-6 | | |
| S51 | A | 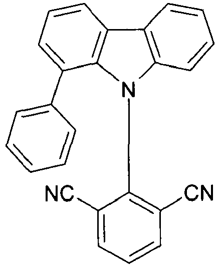 | 13 % |
| | 23039-06-7 | | |
| | 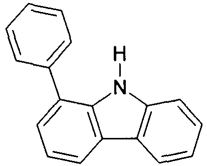 | | |
| | 104636-53-5 | | |
| S52 | A | 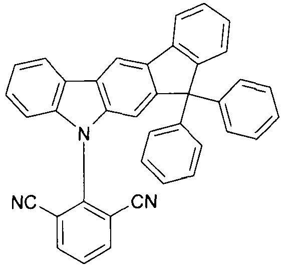 | 32 % |
| | 23039-06-7 | | |
| | 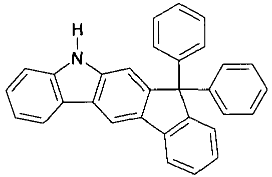 | | |
| | 1257220-52-5 | | |
| S53 | A | 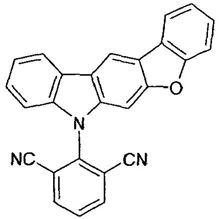 | 34% |
| | 23039-06-7 | | |
| | 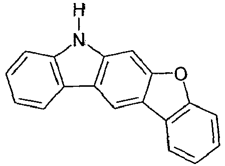 | | |
| | 1246308-83-7 | | |
| S54 | A | 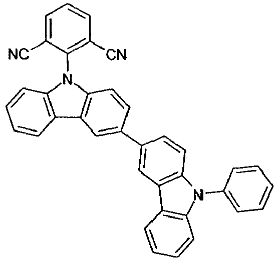 | 28 % |
| | | | |
| | 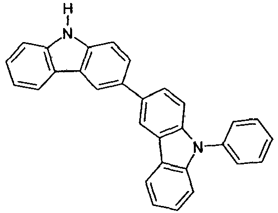 | | |
| | 1060735-14-9 | | |
| S55 | A | 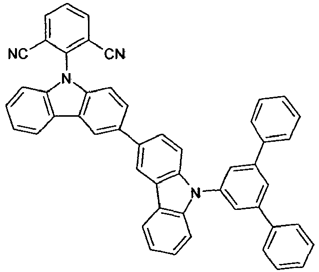 | 33% |
| | 23039-06-7 | | |
| | 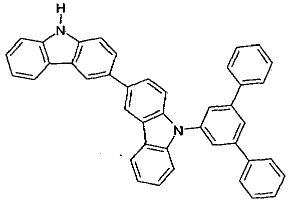 | | |
| | 1415349-61-9 | | |
| S56 | A | 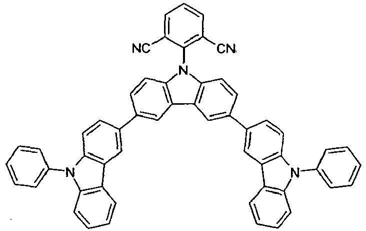 | 28 % |
| | 23039-06-7 | | |
| | 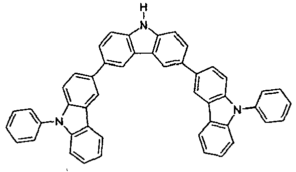 | | |
| | 1060735-19-4 | | |
| S57 | A | 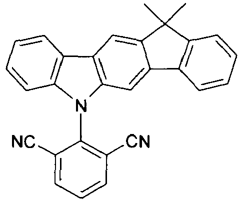 | 30 % |
| | 23039-06-7 | | |
| | 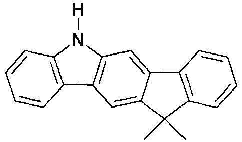 | | |
| | 1260228-95-2 | | |
| S58 | A | 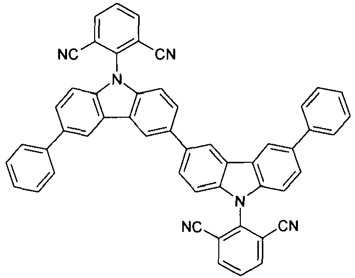 | 18 % |
| | 23039-06-7 | | |
| | 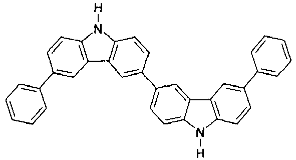 | | |
| | 1303472-75-4 | | |
| S59 # | A | 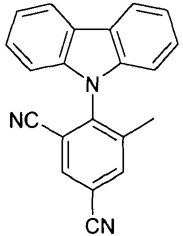 | 16 % |
| | 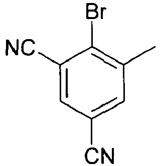 | | |
| | 75344-78-4 | | |
| | 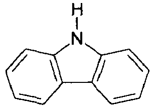 | Es werden - auch im Folgenden - 200 mmol Carbazol-Baustein eingesetzt. | |
| | 51555-21-6 | Es wird 16 h bei 60 °C nachgerührt. | |
| S60 # | A | 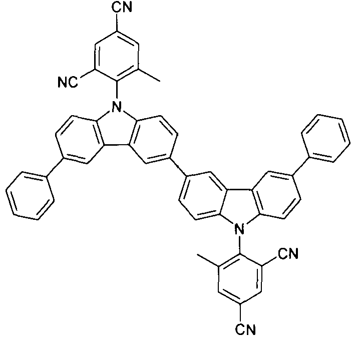 | 22 % |
| | 75344-78-4 | | |
| | 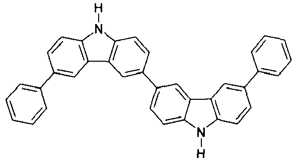 | | |
| | 1303472-75-4 | | |
| S61 # | A | 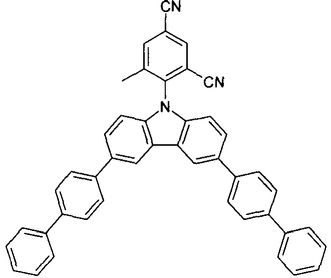 | 21 % |
| | 75344-78-4 | | |
| | 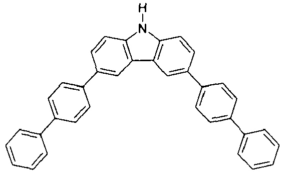 | | |
| | 850264-79-8 | | |
| S62 # | A | 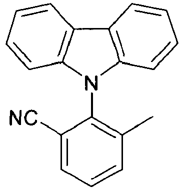 | 28 % |
| | 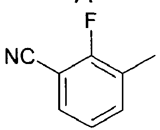 | | |
| | 18547-07-3 | | |
| | 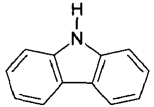 | Es werden - auch im Folgenden - 200 mmol Carbazol-Baustein eingesetzt | |
| | 51555-21-6 | | |
| S63 # | A | 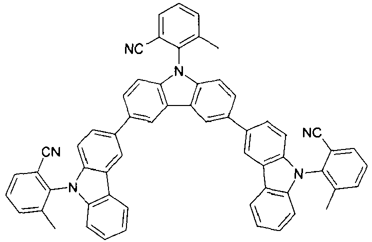 | 13 % |
| | 18547-07-3 | | |
| | 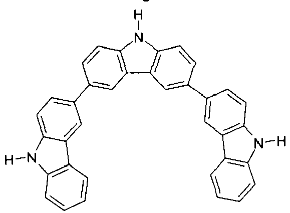 | | |
| | 1346675-22-6 | | |

### Beispiel S64: 1,3,5-Tris-cyano-2-(N-carbazolyl)-4,6-bis-(N-3,6-diphenyl-carbazolyl)benzol, S64 #

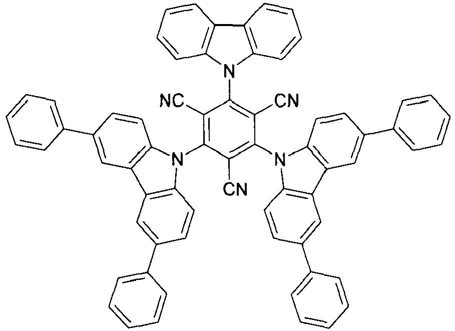

Eine gut gerührte Suspension von 4.0 g (100 mmol) Natriumhydrid, 60 Gew.-% ige Dispersion in Mineralöl, in 500 ml THF wird unter Eiskühlung bei ca. + 10 °C portionsweise mit 16.7 g (100 mmol) Carbazol [51555-21-6] versetzt - Vorsicht Wasserstoffentwicklung! Schäumen! Nach beendeter Zugabe wird die Mischung 30 min. nachgerührt und dann portionsweise unter Eiskühlung so mit 20.7 g (100 mmol) 1,3,5-Tricyano-2-fluor-4,6-dichlor-benzol [25751-93-7] versetzt, dass die Temperatur + 20 °C nicht übersteigt. Nach beendeter Zugabe rührt man 2 h bei + 10 °C nach, entfernt dann das Kältebad, lässt auf 20 - 25 °C erwärmen, rührt 2 h nach und erwärmt dann noch 6 h auf 40 °C. Nach Abkühlen auf Raumtemperatur fügt man 12.0 g (300 mmol) Natriumhydrid, 60 Gew.-% ige Dispersion in Mineralöl zu, kühlt die Reaktionsmischung auf + 10 °C ab, und versetzt dann portionsweise mit 95.8 g (300 mmol) 3,6-Diphenylcarbazol [56525-79-2] - Vorsicht Wasserstoffentwicklung! Schäumen! Nach beendeter Zugabe rührt man 2 h bei + 10 °C nach, entfernt dann das Kältebad, lässt auf 20 - 25 °C erwärmen, rührt 2 h nach und erwärmt dann noch 16 h auf 60 °C. Nach Abkühlen auf Raumtemperatur beendet man die Reaktion durch Zutropfen von 30 ml MeOH und engt die Reaktionsmischung dann im Vakuum fast bis zur Trockene ein. Der Rückstand wird zweimal mit je 600 ml eines Gemischs aus 400 ml Methanol und 200 ml Wasser und dann einmal mit 500 ml Methanol heiß ausgerührt. Die Reinigung erfolgt durch dreimalige Umkristallisation aus Dioxan (ca. 3.5 ml/g) anschließend fünfmalige Umkristallisation aus DMF (ca. 2 ml/g) und zweimalige fraktionierte Sublimation (p ca. 1 x 10⁻⁵ mbar, T ca. 330 - 340 °C). Ausbeute: 22.9 g (24.0 mmol) 24 %. Reinheit: 99.9 % n. HPLC.

Analog werden folgende Verbindungen dargestellt:

| Bsp. | Edukte | Produkt | Ausbeute |
|---|---|---|---|
| S65 # | 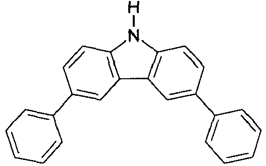 | 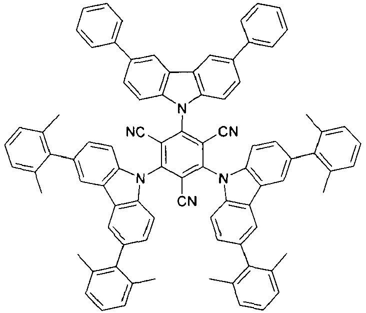 | 20 % |
| | 56525-79-2 | | |
| | 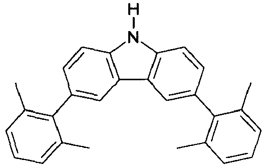 | | |
| | 1246891 | | |
| S66 # | 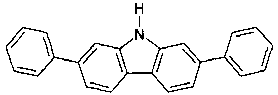 | 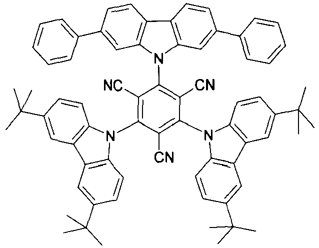 | 19 % |
| | 42448-04-4 | | |
| | 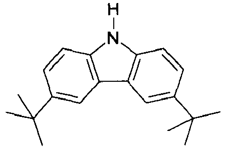 | | |
| | 37500-95-1 | | |
| S67 # | 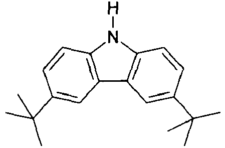 | 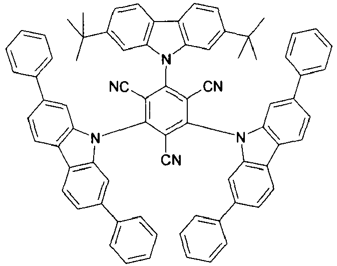 | 21 % |
| | 37500-95-1 | | |
| | 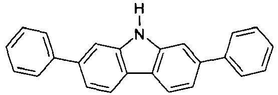 | | |
| | 42448-04-4 | | |
| S68 # | 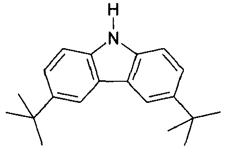 | 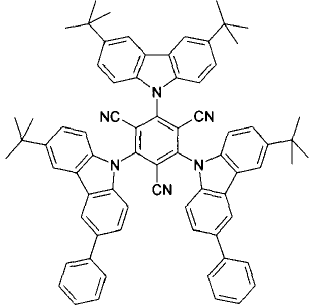 | 24 % |
| | 37500-95-1 | | |
| | 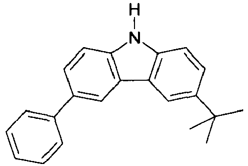 | | |
| | 1335126-51-6 | | |
| S69 # | 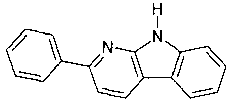 | 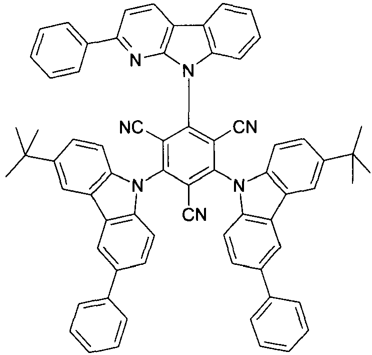 | 17 % |
| | 64677-58-3 | | |
| | 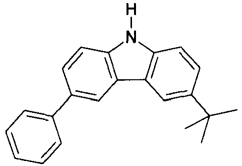 | | |
| | 1335126-51-6 | | |
| S70 # | 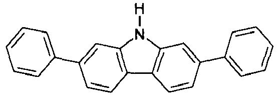 | 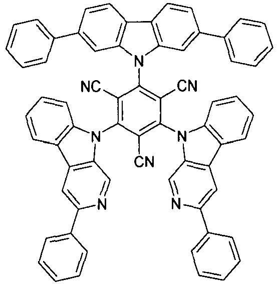 | 18 % |
| | 42448-04-4 | | |
| | 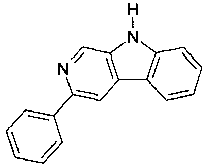 | | |
| | 91944-01-3 | | |
| S71 # | 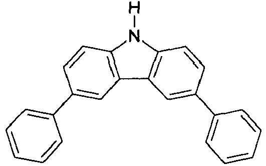 | 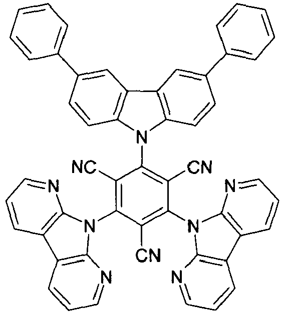 | 20 % |
| | 56525-79-2 | | |
| | 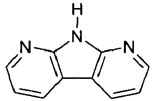 | | |
| | 17966-00-6 | | |

### II) Synthese von Vorstufen

### Beispiel S72-V: 9-(2,6-Dibromo-phenyl)-9H-carbazol

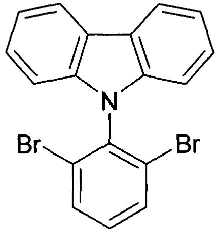

Eine gut gerührte Suspension von 66.9 g (400 mmol) Carbazol [51555-21-6], 25.4 g (100 mmol) 1,3-Dibromo-2-fluorbenzol [363897-9], 106.1 g (500 mmol) Trikaliumphosphat (wasserfrei) und 200 g Glaskugeln wird in 500 ml Dimethylacetamid 16 h bei 160 °C gerührt. Nach Erkalten gibt man 1000 ml Wasser zu, saugt vom ausgefallenen Feststoff ab, wäscht diesen zweimal mit je 300 ml Wasser, zweimal mit je 200 ml Methanol und trocknet dann im Vakuum. Nach einmaliger Umkristallisation aus Toluol/Heptan werden 18.9 g (47.2 mmol, 47 %) erhalten und anschließend weiter umgesetzt.

Analog werden umgesetzt:

| Bsp. | Edukte | Produkt | Ausbeute |
|---|---|---|---|
| S73-V | 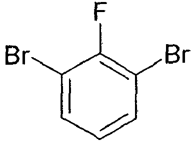 | 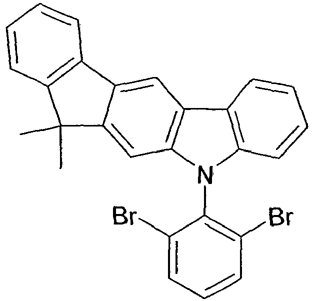 | 28% |
| | 363897-9 | | |
| | 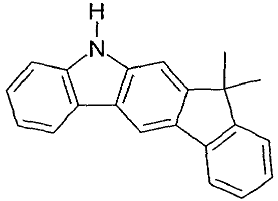 | | |
| | 1.1 Äquivalente 51555-21-6 | | |

### Beispiel S74-V: 9-[2-Bromo-6-(4,4,5,5-tetramethyl-[1,3,2]dioxaborolan--yl)-phenyl]-9H-carbazol

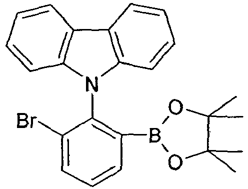

18.5 (46.1 mmol, 1 eq) S72-V wird zusammen mit 14.1 g (55.3 mmol, 1.2 eq) Bis(pinacolato)diboran (CAS 73183-34-3) und 15.8 g (161 mmol, 3.5 eq) Kaliumacetat (CAS 127-08-2) in 100 ml THF vorgelegt und nach Entgasen mit 2.26 g (0.06 eq) 1,1-Bis(diphenylphosphino)ferrocen-dichloropalladium(II)-Komplex mit DCM (CAS 95464-05-4) versetzt. Der Ansatz wird für 14 h am Rückfluss erhitzt und nach beendeter Reaktion wird Wasser zugegeben. Die organische Phase wird abgetrennt und die wässrige Phase mehrfach mit Dichlormethan extrahiert. Die vereinten organischen Phasen werden über Natriumsulfat getrocknet und das Lösungsmittel im Vakuum entfernt. Es werden 15.3 g (34.1 mmol, 74%) des Produktes S74-V erhalten.

Analog werden umgesetzt:

| Bsp. | Edukte | Produkt | Ausbeute |
|---|---|---|---|
| S75-V | 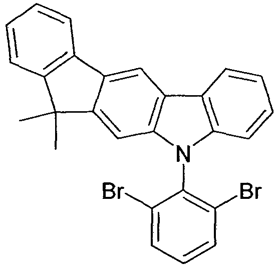 | 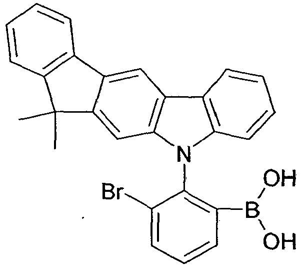 | 67% |

### Beispiel S76-V: 9-[2-Bromo-6-(4,6-diphenyl-[1,3,5]triazin-2-yl)-phenyl]-9H-carbazol S76-V

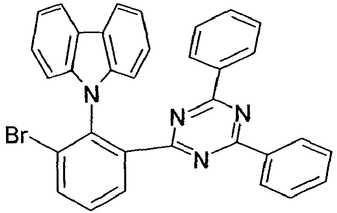

### Variante A

15.0 g (33.5 mmol) S74-V, 11.6 g (43.5 mmol, 1.3 eq 2-Chloro-4,6-diphenyl-1,3,5-triazin (CAS 3842-55-5) und 5.3 g Natriumcarbonat werden in 200 ml Dioxan, 200 ml Toluol und 100 ml Wasser suspendiert. Zu dieser Suspension werden 1.94 g (1.68 mmol, 0.05 eq.) Pd(PPh₃)₄ gegeben. Die Reaktionsmischung über Nacht unter Rückfluss erhitzt. Nach Erkalten wird der ausgefallene Feststoff abgesaugt, mit Wasser und Ethanol gewaschen und getrocknet. Der Rückstand wird mit Toluol heiß extrahiert und aus Toluol/Heptan umkristallisiert. Es werden 7.23 g (13.1 mmol, 39 %) des Produktes S76-V erhalten.

Analog werden umgesetzt:

| Bsp. | Variante Edukte | Produkt | Ausbeute |
|---|---|---|---|
| S77-V | B | 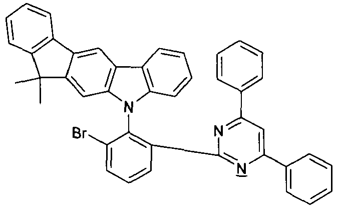 | 39 % |
| | 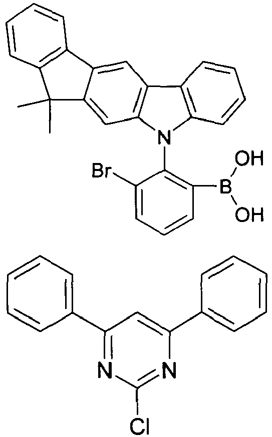 | | |
| | 2915-16-4 | Produktwird nach einmaliger Umkristallisation weiter umgesetzt | |

### III) Synthese von erfindungsgemäßen Verbindungen und Vorstufen:

### Beispiel S78: 9-[3-(4,6-Diphenyl-[1,3,5]triazin-2-yl)-biphenyl-2-yl]-9H-carbazol #

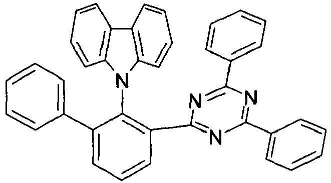

7.1 g (12.8 mmol) S76-V, 1.72 g (14.1 mmol, 1.1 eq) Phenylboronsäure (CAS 98-80-6) und 5.45 g (25.7 mmol, 2 eq) Trikaliumphosphat werden in 100 ml Dioxan, 100 ml Toluol und 50 ml Wasser gelöst und für 30 Minuten entgast. Anschließend werden 86 mg (0.38 mmol, 0.03 eq.) Palladium(II)acetat und 230 mg (0.77 mmol, 0.06eq.) Tri-o-tolylphosphin zugefügt und der Ansatz auf Rückfluss erhitzt. Nach beendeter Reaktion wird der Ansatz abgekühlt, die wässrige Phase abgetrennt und mehrfach mit Toluol extrahiert. Die vereinten organischen Phasen werden mit Wasser gewaschen, über Natriumsulfat getrocknet und das Lösungsmittel im Vakuum entfernt. Der Rückstand wird mit Toluol heiß extrahiert und aus Toluol/Heptan umkristallisiert. Nach Sublimation werden 4.1 g (7.4 mmol, 58 %) des gewünschten Produktes S78 mit einer HPLC-Reinheit >99.9% erhalten.

| Bsp. | Variante Edukte | Produkt | Ausbeute |
|---|---|---|---|
| S79 | B | 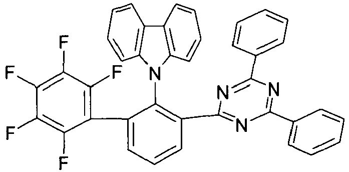 | 37 % |
| | 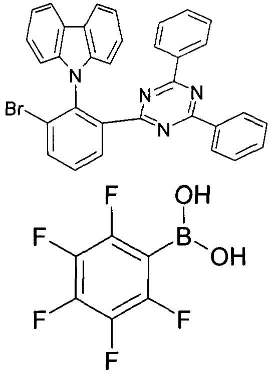 | | |
| | 1582-24-7 | | |
| S80 # | A | 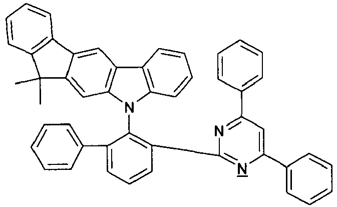 | 64 % |
| | 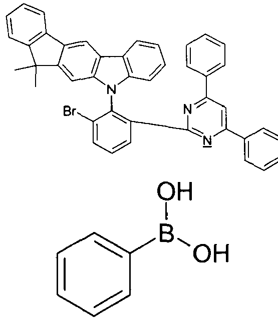 | | |
| | 98-80-6 | | |

### IV) Synthese von Vorstufen:

### Beispiel S81-V:4'-Bromo-2,3,4,5,6-pentafluoro-3',5'-bis-trifluoromethyl-biphenyl

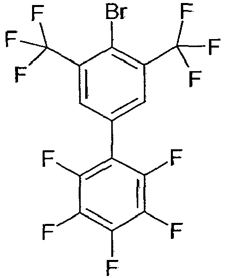

Eine Lösung von 11,4 g (30 mmol) 2,3,4,5,6-Pentafluoro-3',5'-bis-trifluoromethyl-biphenyl [1363958-46-6] in 300 ml Dichlormethan wird unter Lichtausschluss tropfenweise mit einem Gemisch aus 1.7 ml (32 mmol) Brom und 20 ml Chloroform versetzt. Nach 16 h Rühren bei 40 °C gibt man 200 ml Ethanol und dann 50 ml gesättigte Natriumsulfitlösung zu. Der farblose Feststoff wird abgesaugt, dreimal mit 200 ml Wasser und dreimal mit 100 ml Ethanol gewaschen, im Vakuum getrocknet und dann durch Umkristallisieren in DMF und Toluol von Isomeren getrennt. Ausbeute: 4.1 g (9 mmol), 30 % d. Th.

Analog können folgende Verbindungen erhalten werden:

| Bsp. | Edukt 1 | Produkt | Ausbeute |
|---|---|---|---|
| S82-V | 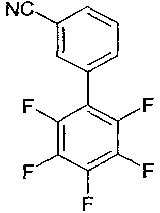 | 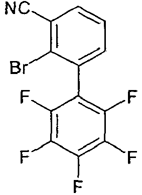 | 32% |
| | [1261665-44-4] | | |
| S83-V | 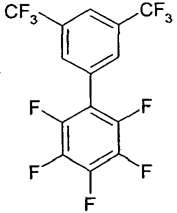 | 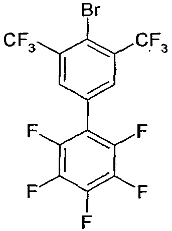 | 69% |
| | [1363958-46-6] | | |
| S84-V | 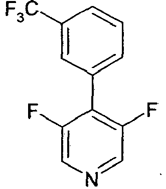 | 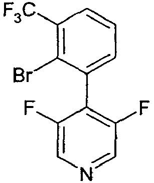 | 31% |
| | [1261886-30-9] | | |
| S85-V | 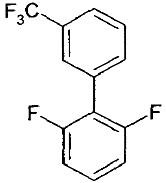 | 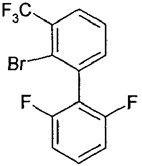 | 27% |
| | [1261805-15-5] | | |
| S86-V | 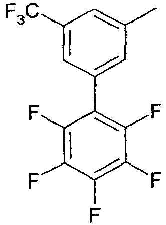 | 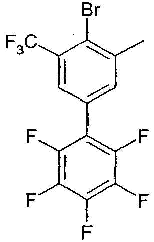 | 55% |
| | [1261494-38-5] | | |
| S87-V | 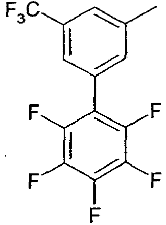 | 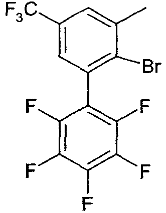 | 30% |
| | [1261494-38-5] | | |
| S88-V | 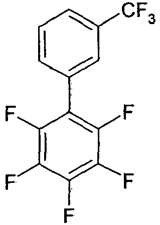 | 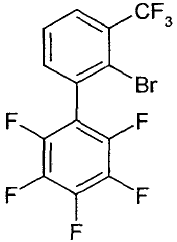 | 29% |
| | [56880-53-6] | | |
| S89-V | 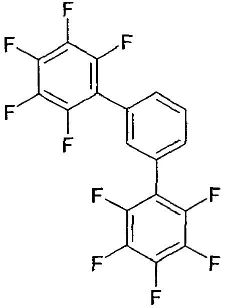 | 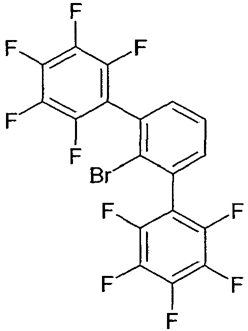 | 27% |
| | [54826-31-2] | | |
| S90-V | 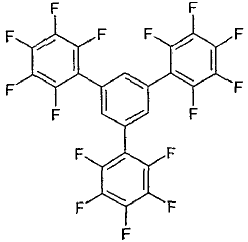 | 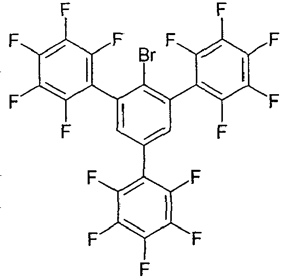 | 37% |
| | [61371-30-0] | | |

### V) Synthese von erfindungsgemäßen Verbindungen:

### Beispiel S91: 9-(2,6-Dimethyl-phenyl)-9H-[3,9']bicarbazolyl

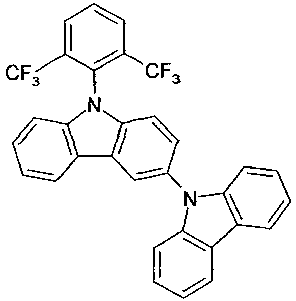

12,6 g (38,32 mmol) 9H-[3,9']Bicarbazolyl [18628-07-4], 7 g (38,32 mmol) 2-Brom-1,3-ditrifluormethyl-benzen [118527-30-3] und 16 g K₂CO₃ werden in 300 mL p-Xylol suspendiert. Zu dieser Suspension werden 0,86 g (3,84 mmol) Pd(OAc)₂ und 7,6 ml einer 1M Tri-tert-butylphosphin-Lösung gegeben. Die Reaktionsmischung wird 16 h unter Rückfluss erhitzt. Nach Erkalten wird die organische Phase abgetrennt, dreimal mit 200 mL Wasser gewaschen und anschließend zur Trockene eingeengt. Der Rückstand wird mit Toluol heiß extrahiert, aus Toluol umkristallisiert und abschließend im Hochvakuum sublimiert. Ausbeute: 14,5 g (35 mmol), 87 % d. Th.; Reiheit 99,9%.

Analog können folgende Verbindungen erhalten werden:

| | Edukt 1 | Edukt 1 | Produkt | Ausbeute |
|---|---|---|---|---|
| S92 | 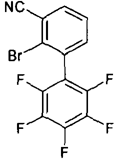 | 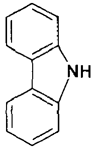 | 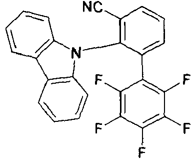 | 77% |
| S93 | 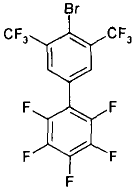 | 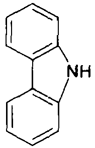 | 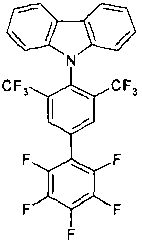 | 68% |
| S94 | 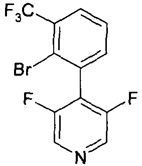 | 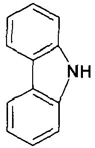 | 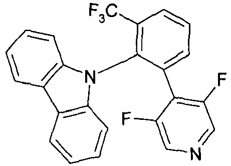 | 76% |
| S95 | 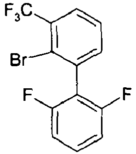 | 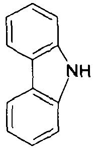 | 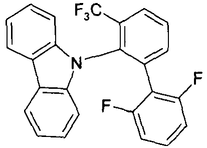 | 69% |
| S96 # | 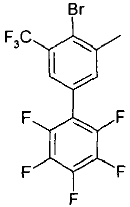 | 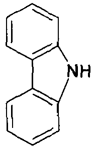 | 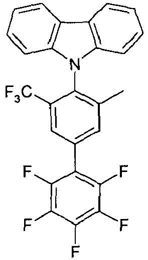 | 81% |
| S97 # | 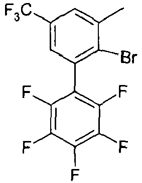 | 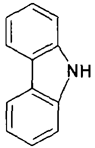 | 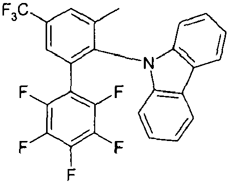 | 63% |
| S98 | 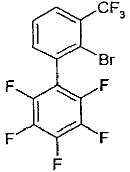 | 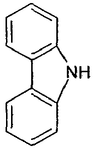 | 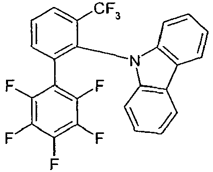 | 59% |
| S99 | 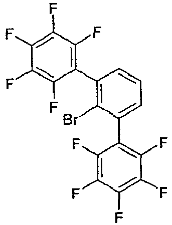 | 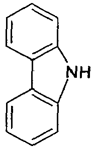 | 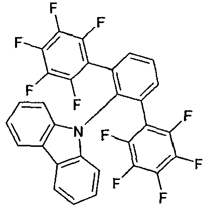 | 52% |
| S100 | 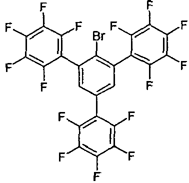 | 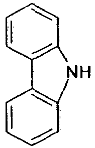 | 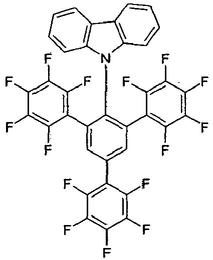 | 48% |
| S101 | 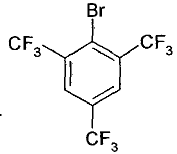 | 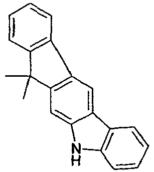 | 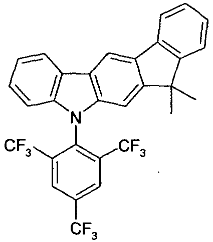 | 79% |
| | [95606-57-8] | [1257220-47-5] | | |
| S102 | 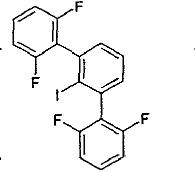 | 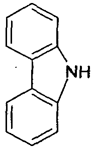 | 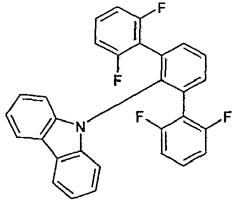 | 74% |
| | [1231208-09-5] | | | |
| S103 | 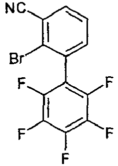 | 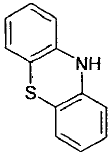 | 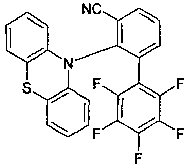 | 76% |
| | | [92-84-2] | | |
| S104 | 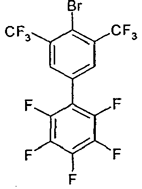 | 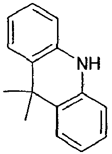 | 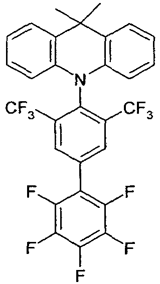 | 71% |
| | | [6267-02-3] | | |
| S105 | 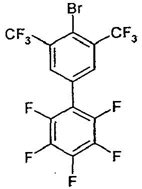 | 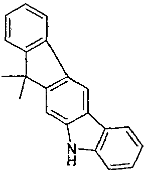 | 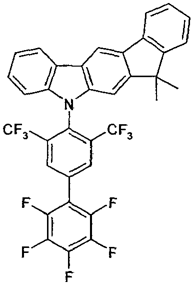 | 75% |
| | | [1257220-47-5] | | |

### VI) Synthese von Vorstufen:

### Beispiel S106-V: 2,3',4,5',6-Pentafluoro-3-tritluoromethyl-biphenyl-2-ylamin

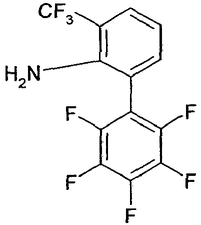

294 ml konzentrierte Salzsäure, 700 ml Ethanol und 38 g (126 mmol) 2,3,4,5,6-Pentafluoro-2'-nitro-3'-trifluoromethyl-biphenyl [1261680-28-7] werden vorgelegt und bei Raumtemperatur portionsweise mit 35 g (294 mmol) Zinn-Pulver versetzt. Nach der Zugabe wird die Mischung 3 h bei Raumtemperatur gerührt. Danach wird die Reaktionsmischung unter Eiskühlung durch Zugabe von NaOH (fest) auf pH = 12 eingestellt. Der Rückstand wird abfiltriert, mit Dichlormethan gewaschen und aus Heptan umkristallisiert. Man erhält 22 g (79 mmol) eines weißen Feststoffes, entsprechend 63 % d. Th.

Analog können folgende Verbindungen mit 2 eq. Zinn-Pulver erhalten werden:

| | Edukt 1 | Produkt | Ausbeute |
|---|---|---|---|
| S107-V | 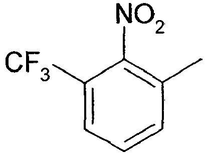 | 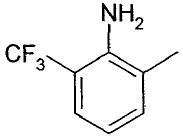 | 61% |
| | [92891-23-1] | | |
| S108-V | 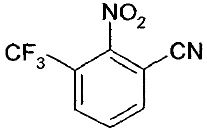 | 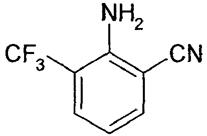 | 68% |
| | [1227572-30-6] | | |

### VII) Synthese von erfindungsgemäßen Verbindungen:

### Beispiel S109: 9-(3,2',3',4',5',6'-Hexafluoro-biphenyl-2-yl)-9H-carbazol

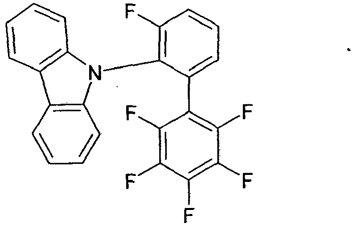

15,6 g (50 mmol) 2,2'-Dibrom-biphenyl werden mit 500 ml Toluen, 2,3 g (2,5 mmol) Tris(dibenzylideneacetone)dipalladium(0), 10 mL 1M t-Bu₃P in Toluol und Natrium-tert-butylat 11,5 g (120 mmol) versetzt. Anschließend werden 11,8 g (40 mmol) 2',3',4',5',6'-Pentafluoro-3-trifluoromethyl-biphenyl-2-ylamin zugegeben. Der Ansatz wird 20 h auf 110 °C erhitzt, dann auf Raumtemperatur abgekühlt und es werden 400 ml Wasser zugegeben. Es wird mit Essigester extrahiert, danach werden die vereinigten organischen Phasen über Natriumsulfat getrocknet, und unter vermindertem Druck eingeengt. Der Rückstand wird aus Toluol und aus Dichlormethan / iso-Propanol umkristallisiert und abschließend im Hochvakuum sublimiert. Die Reinheit beträgt 99,9%. Die Ausbeute beträgt 10 g (23 mmol), entsprechend 59% der Theorie.

Analog können folgende Verbindungen erhalten werden:

| | Edukt 1 | | Produkt | Ausbeute |
|---|---|---|---|---|
| S110 # | 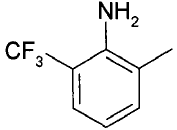 | 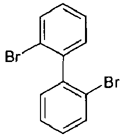 | 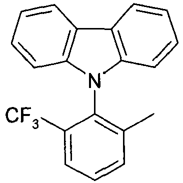 | 67% |
| | [88301-98-8] | [13029-09-9] | | |
| S111 | 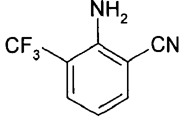 | 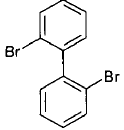 | 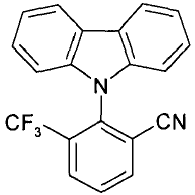 | 67% |
| | [58458-14-3] | [13029-09-9] | | |
| S112 | 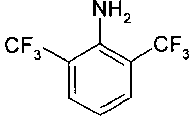 | 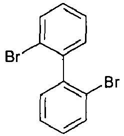 | 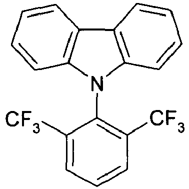 | 59% |
| | [313-13-3] | [13029-09-9] | | |
| S113 # | 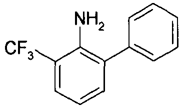 | 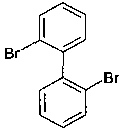 | 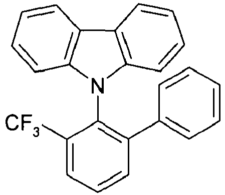 | 58% |
| | [1214363-65-1 ] | [13029-09-9] | | |
| S114 | 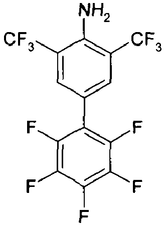 | 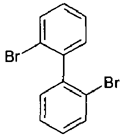 | 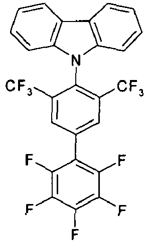 | 68% |
| | | [13029-09-9] | | |
| S115 | 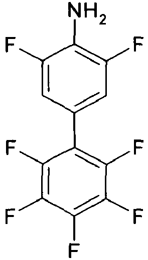 | 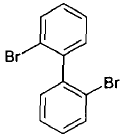 | 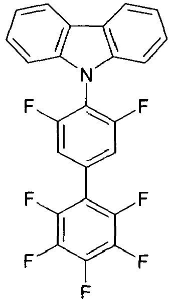 | 63% |
| | [1261616-30-1] | [13029-09-9] | | |
| S116 | 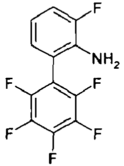 | 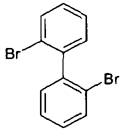 | 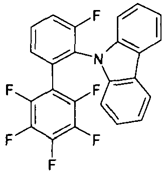 | 55% |
| | [1261759-86.7] | [13029-09-9] | | |
| S117 # | 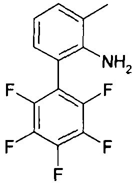 | 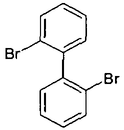 | 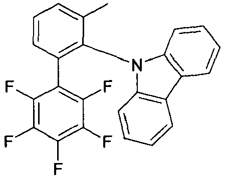 | 46% |
| | [264926-99-0] | [13029-09-9] | | |
| S118 # | 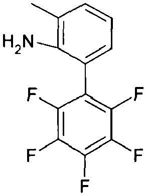 | 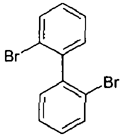 | 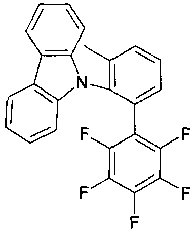 | 52% |
| | [264926-99-0] | [13029-09-9] | | |
| S119 | 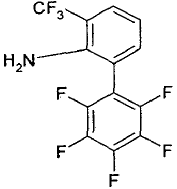 | 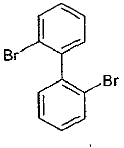 | 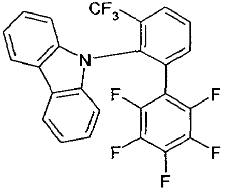 | 43% |
| | | [13029-09-9] | | |

### B) Device-Beispiele

In den folgenden Beispielen werden die Ergebnisse verschiedener OLEDs vorgestellt, bei denen die erfindungsgemäßen Verbindungen als emittierende Verbindungen eingesetzt sind.

Glasplättchen, die mit strukturiertem ITO (Indium Zinn Oxid) beschichtet sind, bilden die Substrate für die OLEDs. Die Substrate werden nass gereinigt (Spülmaschine, Reiniger Merck Extran), anschließend 15 min lang bei 250°C ausgeheizt und vor der Beschichtung mit einem Sauerstoffplasma behandelt.

Alle Materialien werden in einer Vakuumkammer thermisch aufgedampft. Dabei besteht die Emissionsschicht immer aus einem Matrixmaterial und dem emittierenden Material. Dieses wird dem Matrixmaterial durch Coverdampfung in einem bestimmten Volumenanteil beigemischt.

Die OLEDs werden standardmäßig charakterisiert. Die Elektrolumineszenzspektren werden bei einer Leuchtdichte von 1000 cd/m² bestimmt und daraus die CIE 1931 x und y Farbkoordinaten berechnet. Weiterhin wird die Spannung bestimmt, die für eine Leuchtdichte von 1000 cd/m² benötigt wird. In Tabelle 1 ist weiterhin die externe Quanteneffizienz angegeben, die bei einer Betriebsleuchtdichte von 1000 cd/m² erreicht wird. Diese wird unter Annahme einer lambertschen Abstrahlcharakteristik bestimmt.

### Typ1

Substrat:
ITO, 50nm

Lochinjektionsschicht / Lochtransportschicht :
4,4-Bis[N-(1-naphthyl)-N-phenylamino]-biphenyl, α-NPD, [123847-85-8], 90 nm

Emissionschicht:
4,4'-Bis(N-carbazol)biphenyl CBP, [58328-31-7], als Matrixmaterial dotiert mit 5 Vol-% der erfindungsgemäßen Verbindung (s. Tabelle 1 als Dotand,15 nm

Elektronentransportschicht:
1,3,5-Tri(1-pheny)-1H-benzimidazol-2-yl)benzol TPBi, [192198-85-9], 50 nm

Elektroneninjektionsschicht:
LiF,1 nm

Kathode:
Al, 100 nm

### Typ1a

Gleicher Aufbau wie Typ1, mit dem Unterschied, dass eine 120 statt 90 nm dicke Schicht α-NPD und eine 60 statt 50 nm dicke Schicht TPBI verwendet wird.

### Typ2

Substrat:
ITO, 50nm

Lochinjektionsschicht / Lochtransportschicht:
4,4-bis[N-(1-naphthyl)-N-phenylamino]- biphenyl, α-NPD, [123847-85-8], 80 nm

Lochtransportschicht:
1,3-Bis(9-carbazolyl)benzol, mCP, [550378-78-4], 10 nm

Emissionschicht:
2,8-Bis(diphenylphosphoryl)dibenzo[b,d]thiophen, PPT, [1019842-99-9], als Matrixmaterial dotiert mit 5 Vol-% der erfindungsgemäßen Verbindung (s. Tabelle 1) als Dotand, 20 nm

Elektronentransportschicht:
2,8-Bis(diphenylphosphoryl)dibenzo[b,d]thiophen, PPT, [1019842-99-9], 50 nm

Elektroneninjektionsschicht:
LiF,1 nm

Kathode:
Al, 100 nm

### Verwendung von erfindungsgemäßen Verbindungen als Emittermaterialien in OLEDs

Die erfindungsgemäßen Verbindungen lassen sich insbesondere als Emittermaterialien in der Emissionsschicht von OLEDs einsetzen. Die dabei gemessenen Werte für Leistungseffizienz, Spannung und Farbkoordinaten sind in Tabelle 1 zusammengefasst.

**Tabelle 1:**

| **Bsp.** | **Emitter** | **Typ** | **EQE (%) 1000 cd/m²** | **Spannung (V) 1000 cd/m²** | **CIE x/y 1000 cd/m²** |
|---|---|---|---|---|---|
| P1 # | S1 | 2 | 14.0 | 7.3 | 0.42/0.56 |
| P2 # | S6 | 1 | 12.8 | 7.1 | 0.45/0.55 |
| P3 # | S23 | 1 | 12.1 | 5.7 | 0.41/0.57 |
| P4 # | S32 | 1 | 13.0 | 6.3 | 0.36/0.59 |
| P5 # | S36 | 2 | 14.4 | 7.7 | 0.40/0.57 |
| P6 # | S47 | 2 | 15.5 | 8.3 | 0.14/0.19 |
| P7 # | S49 | 1 | 18.5 | 5.8 | 0.23/0.54 |
| P8 | S52 | 2 | 16.8 | 6.6 | 0.15/0.28 |
| P9 | S54 | 1 | 17.2 | 4.7 | 0.22/0.52 |
| P10 # | S61 | 2 | 13.8 | 7.6 | 0.15/0.27 |
| P11 # | S64 | 1a | 13.8 | 6.3 | 0.59/0.41 |
| P-V1 | Vgl.1 | 2 | 1.8 | 4.6 | 0.14/0.06 |
| P-V2 | Vgl.2 | 1 | 1.3 | 3.7 | 0.69/0.31 |

Die hergestellten OLEDs zeigen hervorragende Werte für die Leistungseffizienz. Dabei ist durch Verwendung unterschiedlicher Emitter das Erhalten von Licht mit unterschiedlichen Farbkoordinaten möglich. Der Vergleich mit Verbindungen gemäß dem Stand der Technik (Vgl.1 und Vgl. 2 in P-V1 und P-V2) zeigt eine überragende Verbesserung der Leistungseffizienz bei ähnlichen Spannungen durch Ersetzen dieser Verbindungen durch die erfindungsgemäßen Verbindungen.

Verbindungen der Vergleichsbeispiele P-1 und P-V2:

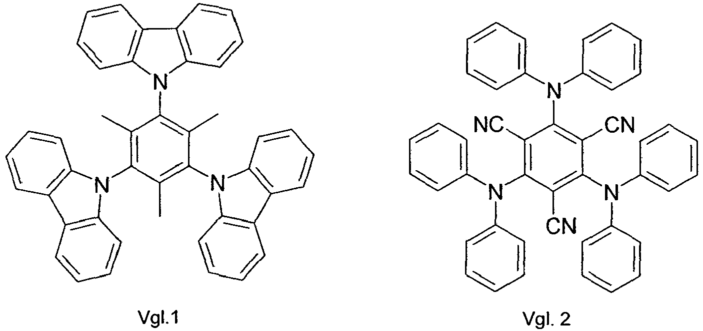

## Patentansprüche

1. Verbindung der Formel (I) 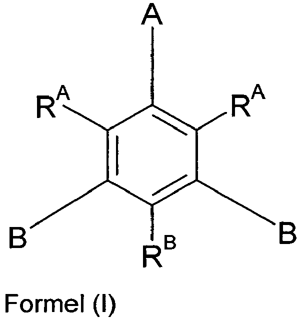 wobei gilt:
A ist eine Gruppe der Formel (A) 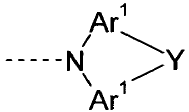 Formel (A), die über die gestrichelte Bindung gebunden ist;
Ar¹ ist bei jedem Auftreten gleich oder verschieden ein aromatisches oder heteroaromatisches Ringsystem mit 5 bis 30 aromatischen Ringatomen, das mit einem oder mehreren Resten R¹ substituiert sein kann;
Y ist bei jedem Auftreten gleich oder verschieden eine Einfachbindung, BR¹, C(R¹)₂, Si(R¹)₂, NR¹, PR¹, P(=O)R¹, O, S, S=O oder S(=O)₂;
B ist bei jedem Auftreten gleich oder verschieden gewählt aus H, einer geradkettigen Alkylgruppe mit 1 bis 10 C-Atomen, einer verzweigten oder cyclischen Alkylgruppe mit 3 bis 10 C-Atomen, die jeweils mit einem oder mehreren Resten R¹ substituiert sein können, und einer Arylgruppe mit 6 bis 14 aromatischen Ringatomen, die jeweils mit einem oder mehreren Resten R¹ substituiert sein kann;
R^{A} ist bei jedem Auftreten gleich oder verschieden F, CF₃, CN, oder eine Gruppe E, die eine Aryl- oder Heteroarylgruppe mit 6 bis 14 aromatischen Ringatomen ist, die mit einem oder mehreren Resten R¹ substituiert sein kann, und die als Bestandteile des aromatischen Rings eine oder mehrere Gruppen V enthält, wobei die Gruppen V bei jedem Auftreten gleich oder verschieden gewählt sind aus =N-, =C(F)-, =C(CN)- und =C(CF₃)-, und wobei die Heteroarylgruppe nicht über ein Stickstoffatom gebunden ist;
R^{B} ist ausgewählt aus H, einer geradkettigen Alkylgruppe mit 1 bis 10 C-Atomen oder einer verzweigten oder cyclischen Alkylgruppe mit 3 bis 10 C-Atomen, die jeweils mit einem oder mehreren Resten R¹ substituiert sein können, und einer Arylgruppe mit 6 bis 14 aromatischen Ringatomen, die mit einem oder mehreren Resten R¹ substituiert sein kann;
R¹ ist bei jedem Auftreten gleich oder verschieden H, D, F, C(=O)R², CN, Si(R²)₃, N(R²)₂, P(=O)(R²)₂, OR², S(=O)R², S(=O)₂R², eine geradkettige Alkyl- oder Alkoxygruppe mit 1 bis 20 C-Atomen oder eine verzweigte oder cyclische Alkyl-oder Alkoxygruppe mit 3 bis 20 C-Atomen, wobei die oben genannten Gruppen jeweils mit einem oder mehreren Resten R² substituiert sein können und wobei eine oder mehrere CH₂-Gruppen in den oben genannten Gruppen durch-R²C=CR²-, -C≡C-, Si(R²)₂, C=O, C=NR², -C(=0)0-, -C(=O)NR²-, NR², P(=O)(R²), -O-, -S-, SO oder SO₂ ersetzt sein können, oder ein aromatisches oder heteroaromatisches Ringsystem mit 5 bis 30 aromatischen Ringatomen, das jeweils durch einen oder mehrere Reste R² substituiert sein kann, wobei zwei oder mehr Reste R¹ miteinander verknüpft sein können und einen Ring bilden können;
R² ist bei jedem Auftreten gleich oder verschieden H, D, F oder ein aliphatischer, aromatischer oder heteroaromatischer organischer Rest mit 1 bis 20 C-Atomen, in dem auch ein oder mehrere H-Atome durch D oder F ersetzt sein können; dabei können zwei oder mehr Substituenten R² miteinander verknüpft sein und einen Ring bilden;
wobei die folgenden Verbindungen ausgenommen sind:

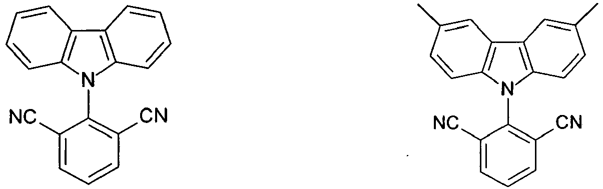

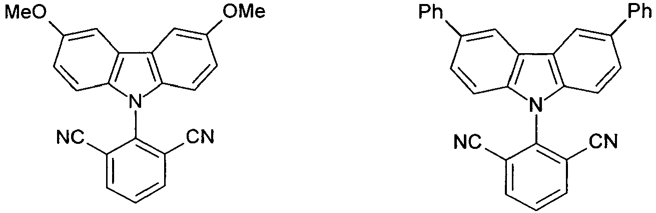

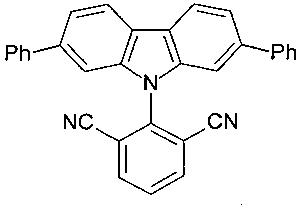

2. Verbindung nach Anspruch 1, **dadurch gekennzeichnet, dass** Ar¹ bei jedem Auftreten gleich oder verschieden ein aromatisches Ringsystem mit 6 bis 20 aromatischen Ringatomen ist, das mit einem oder mehreren Resten R¹ substituiert sein kann.

3. Verbindung nach Anspruch 1 oder 2, **dadurch gekennzeichnet, dass** die Gruppe Y bei jedem Auftreten gleich oder verschieden eine Einfachbindung, C(R¹)₂, NR¹, O oder S ist.

4. Verbindung nach einem oder mehreren der Ansprüche 1 bis 3,
**dadurch gekennzeichnet, dass** die Gruppe B gleich H ist.

5. Verbindung nach einem oder mehreren der Ansprüche 1 bis 4,
**dadurch gekennzeichnet, dass** beide Reste R^{A} gleich CN sind.

6. Verbindung nach einem oder mehreren der Ansprüche 1 bis 5,
**dadurch gekennzeichnet, dass** R^{B} gleich H ist.

7. Verbindung nach einem oder mehreren der Ansprüche 1 bis 6,
**dadurch gekennzeichnet, dass** die Verbindung der Formel (I) der Formel (I-3) entspricht 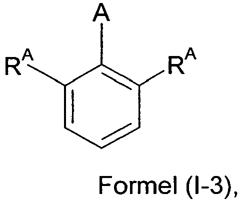 wobei die Gruppen A und R^{A} wie in einem oder mehreren der Ansprüche 1 bis 6 definiert sind.

8. Verfahren zur Herstellung einer Verbindung gemäß Formel (I) nach einem oder mehreren der Ansprüche 1 bis 7, **dadurch gekennzeichnet, dass** mindestens ein Carbazolderivat durch nukleophile aromatische Substitution oder Buchwald-Kupplung eingeführt wird, oder dass mindestens eine elektronenarme Heteroarylgruppe durch Suzuki-Kupplung eingeführt wird.

9. Oligomere, enthaltend eine oder mehrere Verbindungen gemäß Formel (I) nach einem oder mehreren der Ansprüche 1 bis 7, wobei die Bindung(en) zum Oligomer an beliebigen, in Formel (I) mit R¹ oder R² substituierten Positionen lokalisiert sein können.

10. Elektronische Vorrichtung, enthaltend mindestens eine Verbindung nach einem oder mehreren der Ansprüche 1 bis 7, oder mindestens ein Oligomer nach Anspruch 9.

11. Organische Elektrolumineszenzvorrichtung, enthaltend Anode, Kathode und mindestens eine emittierende Schicht, **dadurch gekennzeichnet, dass** mindestens eine organische Schicht in der Vorrichtung, gewählt aus emittierenden Schichten, mindestens eine Verbindung nach einem oder mehreren der Ansprüche 1 bis 7 oder mindestens ein Oligomer nach Anspruch 9 enthält.

12. Verwendung einer Verbindung nach einem oder mehreren der Ansprüche 1 bis 7, oder eines Oligomers nach Anspruch 9, in einer elektronischen Vorrichtung.

## Claims

1. Compound of the formula (I) 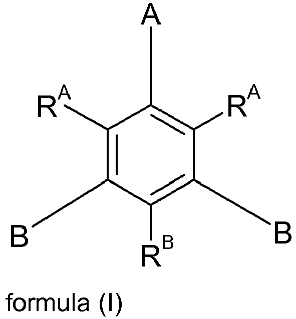 where:
A is a group of the formula (A) 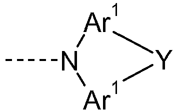 formula (A), which is bonded via the dashed bond;
Ar¹ is on each occurrence, identically or differently, an aromatic or heteroaromatic ring system having 5 to 30 aromatic ring atoms, which may be substituted by one or more radicals R¹;
Y is on each occurrence, identically or differently, a single bond, BR¹, C(R¹)₂, Si(R¹)₂, NR¹, PR¹, P(=O)R¹, O, S, S=O oder S(=O)₂;
B is selected on each occurrence, identically or differently, from H, a straight-chain alkyl group having 1 to 10 C atoms, a branched or cyclic alkyl group having 3 to 10 C atoms, which may in each case be substituted by one or more radicals R¹, and an aryl group having 6 to 14 aromatic ring atoms, which may in each case be substituted by one or more radicals R¹;
R^{A} is on each occurrence, identically or differently, F, CF₃, CN, or a group E, which is an aryl or heteroaryl group having 6 to 14 aromatic ring atoms, which may be substituted by one or more radicals R¹, and which contains, as constituents of the aromatic ring, one or more groups V, where the groups V are selected on each occurrence, identically or differently, from =N-, =C(F)-, =C(CN)- and =C(CF₃)-, and where the heteroaryl group is not bonded via a nitrogen atom;
R^{B} is selected from H, a straight-chain alkyl group having 1 to 10 C atoms or a branched or cyclic alkyl group having 3 to 10 C atoms, which may in each case be substituted by one or more radicals R¹, and an aryl group having 6 to 14 aromatic ring atoms, which may be substituted by one or more radicals R¹;
R¹ is on each occurrence, identically or differently, H, D, F, C(=O)R², CN, Si(R²)₃, N(R²)₂, P(=O)(R²)₂, OR², S(=O)R², S(=O)₂R², a straight-chain alkyl or alkoxy group having 1 to 20 C atoms or a branched or cyclic alkyl or alkoxy group hav-ing 3 to 20 C atoms, where the above-mentioned groups may in each case be substituted by one or more radicals R² and where one or more CH₂ groups in the above-mentioned groups may be replaced by -R²C=CR²-, -C=C-, Si(R²)₂, C=O, C=NR², -C(=O)O-, -C(=O)NR²-, NR², P(=O)(R²), -O-, -S-, SO or SO₂, or an aromatic or heteroaromatic ring system having 5 to 30 aromatic ring atoms, which may in each case be substituted by one or more radicals R², where two or more radicals R¹ may be linked to one another and may form a ring;
R² is on each occurrence, identically or differently, H, D, F or an aliphatic, aromatic or heteroaromatic organic radical having 1 to 20 C atoms, in which, in addition, one or more H atoms may be replaced by D or F; two or more substituents R² here may be linked to one another and may form a ring;
where the following compounds are excluded:

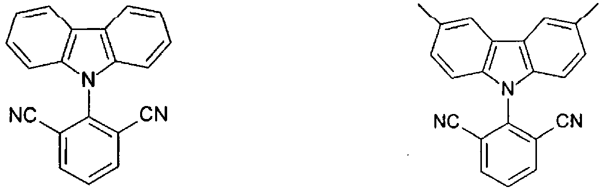

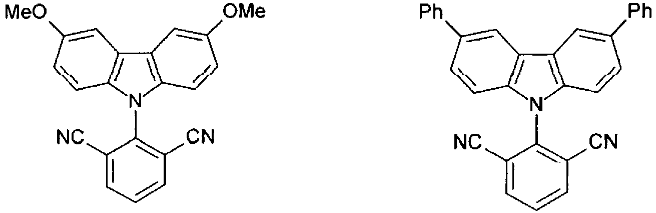

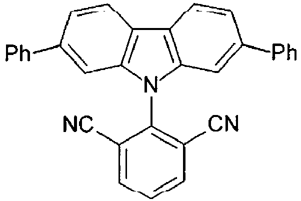

2. Compound according to Claim 1, **characterised in that** Ar¹ is on each occurrence, identically or differently, an aromatic ring system having 6 to 20 aromatic ring atoms, which may be substituted by one or more radicals R¹.

3. Compound according to Claim 1 or 2, **characterised in that** the group Y is on each occurrence, identically or differently, a single bond, C(R¹)₂, NR¹, O or S.

4. Compound according to one or more of Claims 1 to 3, **characterised in that** the group B is equal to H.

5. Compound according to one or more of Claims 1 to 4, **characterised in that** both radicals R^{A} are equal to CN.

6. Compound according to one or more of Claims 1 to 5, **characterised in that** R^{B} is equal to H.

7. Compound according to one or more of Claims 1 to 6, **characterised in that** the compound of the formula (I) corresponds to the formula (I-3) 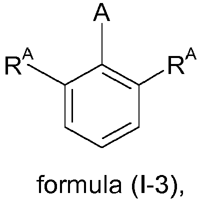 where the groups A and R^{A} are defined as in one or more of Claims 1 to 6.

8. Process for the preparation of a compound of the formula (I) according to one or more of Claims 1 to 7, **characterised in that** at least one carbazole derivative is introduced by nucleophilic aromatic substitution or Buchwald coupling, or **in that** at least one electron-deficient heteroaryl group is introduced by Suzuki coupling.

9. Oligomers containing one or more compounds of the formula (I) according to one or more of Claims 1 to 7, where the bond(s) to the oligomer may be localised at any desired positions in formula (I) that are substituted by R¹ or R².

10. Electronic device containing at least one compound according to one or more of Claims 1 to 7, or at least one oligomer according to Claim 9.

11. Organic electroluminescent device comprising anode, cathode and at least one emitting layer, **characterised in that** at least one organic layer in the device, selected from emitting layers, comprises at least one compound according to one or more of Claims 1 to 7 or at least one oligomer according to Claim 9.

12. Use of a compound according to one or more of Claims 1 to 7 or of an oligomer according to Claim 9 in an electronic device.

## Revendications

1. Composé de la formule (I) : 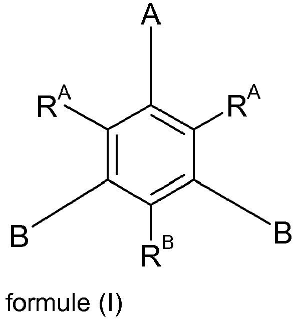 dans laquelle :
A est un groupe de la formule (A) : 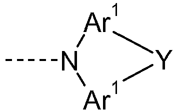 formule (A), qui est lié via la liaison en pointillés ;
Ar¹ est pour chaque occurrence, de manière identique ou différente, un système de cycle aromatique ou hétéroaromatique qui comporte de 5 à 30 atomes de cycle aromatique, lequel peut être substitué par un radical ou par plusieurs radicaux R¹ ;
Y est pour chaque occurrence, de manière identique ou différente, une liaison simple, BR¹, C(R¹)₂, Si(R¹)₂, NR¹, PR¹, P(=O)R¹, O, S, S=O ou S(=O)₂ ;
B est sélectionné pour chaque occurrence, de manière identique ou différente, parmi H, un groupe alkyle en chaîne droite qui comporte de 1 à 10 atome(s) de C, un groupe alkyle ramifié ou cyclique qui comporte de 3 à 10 atomes de C, lequel peut dans chaque cas être substitué par un radical ou par plusieurs radicaux R¹, et un groupe aryle qui comporte de 6 à 14 atomes de cycle aromatique, lequel peut dans chaque cas être substitué par un radical ou par plusieurs radicaux R¹ ;
R^{A} est pour chaque occurrence, de manière identique ou différente, F, CF₃, CN, ou un groupe E, lequel est un groupe aryle ou hétéroaryle qui comporte de 6 à 14 atomes de cycle aromatique, lequel peut être substitué par un radical ou par plusieurs radicaux R¹, et lequel contient, en tant que constituants du cycle aromatique, un ou plusieurs groupe(s) V, où les groupes V sont sélectionnés pour chaque occurrence, de manière identique ou différente, parmi =N-, =C(F)-, =C(CN)et =C(CF₃)-, et où le groupe hétéroaryle n'est pas lié via un atome d'azote ;
R^{B} est sélectionné parmi H, un groupe alkyle en chaîne droite qui comporte de 1 à 10 atome(s) de C ou un groupe alkyle ramifié ou cyclique qui comporte de 3 à 10 atomes de C, lequel peut dans chaque cas être substitué par un radical ou par plusieurs radicaux R¹, et un groupe aryle qui comporte de 6 à 14 atomes de cycle aromatique, lequel peut être substitué par un radical ou par plusieurs radicaux R¹ ;
R¹ est pour chaque occurrence, de manière identique ou différente, H, D, F, C(=O)R², CN, Si(R²)₃, N(R²)₂, P(=O)(R²)₂, OR², S(=O)R², S(=O)₂R², un groupe alkyle ou alcoxy en chaîne droite qui comporte de 1 à 20 atome(s) de C ou un groupe alkyle ou alcoxy ramifié ou cyclique qui comporte de 3 à 20 atomes de C, où les groupes qui ont été mentionnés ciavant peuvent dans chaque cas être substitués par un radical ou par plusieurs radicaux R² et où un ou plusieurs groupe(s) CH₂ dans les groupes qui ont été mentionnés ci-avant peut/ peuvent être remplacé(s) par -R²C=CR²-, -C=C-, Si(R²)₂, C=O, C=NR², -C(=O)O-, -C(=O)NR²-, NR², P(=O)(R²), -O-, -S-, SO ou SO₂, ou un système de cycle aromatique ou hétéroaromatique qui comporte de 5 à 30 atomes de cycle aromatique, lequel peut dans chaque cas être substitué par un radical ou par plusieurs radicaux R², où deux radicaux R¹ ou plus peuvent être liés l'un à l'autre ou les uns aux autres et peuvent former un cycle ;
R² est pour chaque occurrence, de manière identique ou différente, H, D, F ou un radical organique aliphatique, aromatique ou hétéroaromatique qui comporte de 1 à 20 atome(s) de C, où, en outre, un ou plusieurs atome(s) de H peut/ peuvent être remplacé(s) par D ou F ; deux substituants R² ou plus peuvent ici être liés l'un à l'autre ou les uns aux autres et peuvent former un cycle ;
où les composés qui suivent sont exclus :

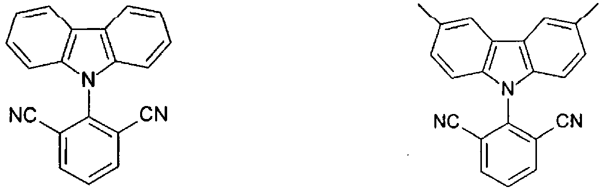

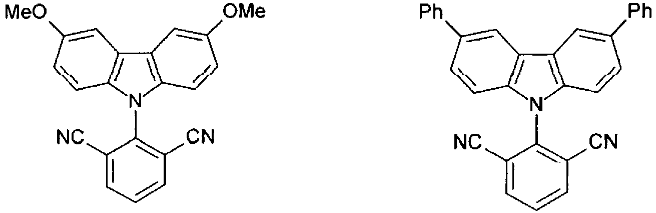

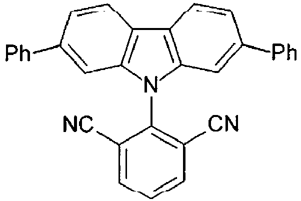

2. Composé selon la revendication 1, **caractérisé en ce que** Ar¹ est pour chaque occurrence, de manière identique ou différente, un système de cycle aromatique qui comporte de 6 à 20 atomes de cycle aromatique, lequel peut être substitué par un radical ou par plusieurs radicaux R¹.

3. Composé selon la revendication 1 ou 2, **caractérisé en ce que** le groupe Y est pour chaque occurrence, de manière identique ou différente, une liaison simple, C(R¹)₂, NR¹, O ou S.

4. Composé selon une ou plusieurs des revendications 1 à 3, **caractérisé en ce que** le groupe B est égal à H.

5. Composé selon une ou plusieurs des revendications 1 à 4, **caractérisé en ce que** les deux radicaux R^{A} sont égaux à CN.

6. Composé selon une ou plusieurs des revendications 1 à 5, **caractérisé en ce que** R^{B} est égal à H.

7. Composé selon une ou plusieurs des revendications 1 à 6, **caractérisé en ce que** le composé de la formule (I) correspond à la formule (I-3) : 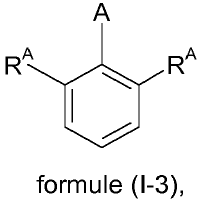 dans laquelle les groupes A et R^{A} sont définis tel que selon une ou plusieurs des revendications 1 à 6.

8. Procédé pour la préparation d'un composé de la formule (I) selon une ou plusieurs des revendications 1 à 7, **caractérisé en ce qu'**au moins un dérivé carbazole est introduit au moyen d'une substitution aromatique nucléophile ou d'un couplage de Buchwald, ou **en ce qu'**au moins un groupe hétéroaryle déficient en électrons est introduit au moyen d'un couplage de Suzuki.

9. Oligomères contenant un ou plusieurs composé(s) de la formule (I) selon une ou plusieurs des revendications 1 à 7, où la/les liaison(s) sur l'oligomère peut/peuvent être localisée(s) au niveau de n'importe quelles positions souhaitées dans la formule (I) qui sont substituées par R¹ ou par R².

10. Dispositif électronique contenant au moins un composé selon une ou plusieurs des revendications 1 à 7, ou au moins un oligomère selon la revendication 9.

11. Dispositif électroluminescent organique comprenant une anode, une cathode et au moins une couche d'émission, **caractérisé en ce qu'**au moins une couche organique dans le dispositif, qui est sélectionnée parmi des couches d'émission, comprend au moins un composé selon une ou plusieurs des revendications 1 à 7 ou au moins un oligomère selon la revendication 9.

12. Utilisation d'un composé selon une ou plusieurs des revendications 1 à 7 ou d'un oligomère selon la revendication 9 dans un dispositif électronique.
